(19) Europäisches Patentamt / European Patent Office / Office européen des brevets

(11) **EP 3 394 777 B1**

(12) **EUROPEAN PATENT SPECIFICATION**

(45) Date of publication and mention
of the grant of the patent:
**24.07.2024   Bulletin 2024/30**

(21) Application number: **16836098.0**

(22) Date of filing: **23.12.2016**

(51) International Patent Classification (IPC):
**G16B 25/20** $^{(2019.01)}$

(52) Cooperative Patent Classification (CPC):
**G16B 25/00; G16B 45/00; G16B 25/20;
G16B 40/00**

(86) International application number:
**PCT/IB2016/057970**

(87) International publication number:
**WO 2017/109762 (29.06.2017 Gazette 2017/26)**

(54) **COMPUTER-BASED METHOD FOR DESIGNING A SET OF PRIMERS**

COMPUTERBASIERTES VERFAHREN ZUM ENTWURF EINES PRIMER-SETS

PROCÉDÉ INFORMATIQUE POUR CONCEVOIR UN ENSEMBLE D'AMORCES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.12.2015   IT UB20159170**

(43) Date of publication of application:
**31.10.2018   Bulletin 2018/44**

(73) Proprietor: **DiaSorin Italia S.p.A.
13040 Saluggia (Vercelli) (IT)**

(72) Inventor: **RUO RUI, Luciano
13040 Saluggia Vercelli (IT)**

(74) Representative: **Chimini, Francesco
Jacobacci & Partners S.p.A.
Piazza della Vittoria 11
25122 Brescia (IT)**

(56) References cited:
• **"Protein Electrophoresis", vol. 402, 1 January
2007, NEW YORK ; HEIDELBERG [U.A.] :
HUMANA PRESS,, US, ISBN: 978-1-60761-961-1,
ISSN: 1064-3745, article SANTALUCIA JOHN:
"Physical Principles and Visual-OMP Software
for Optimal PCR Design", pages: 3 - 33,
XP055849549, DOI: 10.1007/978-1-59745-528-2_1**

• **EIKEN: "A Guide to LAMP primer designing", 20
August 2006 (2006-08-20), XP055300394,
Retrieved from the Internet
<URL:https://primerexplorer.jp/e/v4_manual/pdf
/PrimerExplorerV4_Manual_1.pdf> [retrieved on
20160906]**
• **EIKEN: "How to use PrimerExplorer V4", 24
November 2015 (2015-11-24), XP055300395,
Retrieved from the Internet
<URL:https://primerexplorer.jp/e/v4_manual/pdf
/PrimerExplorerV4_Manual_2.pdf> [retrieved on
20160906]**
• **ANONYMOUS: "LAMP primer designing software
PrimerExplorer- Appendix", 24 November 2015
(2015-11-24), XP055300457, Retrieved from the
Internet
<URL:https://primerexplorer.jp/e/v4_manual/03.
html> [retrieved on 20160907]**
• **ANONYMOUS: "LAMP primer designing software
PrimerExplorer- release version 4", 24 November
2015 (2015-11-24), XP055300455, Retrieved from
the Internet <URL:https://primerexplorer.jp/e/>
[retrieved on 20160907]**
• **Y. KIMURA ET AL: "Optimization of turn-back
primers in isothermal amplification", NUCLEIC
ACIDS RESEARCH, vol. 39, no. 9, 1 May 2011
(2011-05-01), pages e59 - e59, XP055164865,
ISSN: 0305-1048, DOI: 10.1093/nar/gkr041**

**Description**

The present invention relates to a computer-based method for designing nucleic acid molecules, in particular nucleic acids sequences (oligonucleotides) called "primers". The invention allows to design, analyze and evaluate the nucleic acid molecules for particular uses or applications, in particular the design of primers to be used with the LAMP technique (Loop-mediated isothermal amplification).

The invention also relates to a computer and a computer program product suitable to implement this method.

The improvement of the efficiency of DNA amplification techniques (such as PCR, LAMP, etc.) is reached through the design and, in particular, the optimization of the hybridization or base pairing between sequences of nucleic acids. The accurate prediction of the thermodynamic parameters allows an optimal choice of the sequences and of the reaction conditions (temperature and salt concentrations).

The LAMP technique (T. Notomi, H. Okayama, H. Masubuchi et al., "Loop-mediated isothermal amplification of DNA," Nucleic Acids Research, vol. 28, no. 12, article e63, 2000) is a technique for the amplification and detection of nucleic acids: this method can amplify large amount of genomic material in isothermal conditions with high specificity, efficiency and speed.

This technique is based on using a DNA polymerase and a set of 4 fundamental primers (2 external primers, F3 and B3, and 2 internal primer, FIP and BIP, constituted by two distinct nucleotidic regions respectively), which guarantee the formation of the structure called "dumbbell" (shaped like a "dumbbell", the piece of equipment used in weight training, of its graphical representation), the starting point of the exponential isothermal amplification reaction. Furthermore two additional primers, called Loop Primers (LF and LB) can be used together with the base set, by acting as catalysts of the amplification reaction. Consequently, the design of primers for a LAMP assay requires the selection of 6-8 different nucleotidic sequences within the genomic region of interest, giving high specificity to this technique.

A careful selection of the position of the primers and of their melting temperature (Tm) is critical to achieve the reaction and to obtain the hybridization, properly synchronized, of the different primers on the target.

For this reason, the primer design process is a crucial step in developing a LAMP assay.

The melting temperature Tm is a very important parameter for the design. It corresponds to the temperature in which the strand of the target sequence is hybridized, for the 50%, to the primer. In other words, at this temperature, one half of the strands is in the form a double helix, while the other half is in the denatured state ("random coil").

At the moment, there are just a few LAMP primer design methods. The main reference is the PrimerExplorer software program, supplied by Eiken Chemical Co. Once provided the target sequence and a number of design parameters, this program processes and displays the most efficient primer sets, according to its algorithm. PrimerExplorer however has some limitations.

In PrimerExplorer, the design of loop primers requires an additional session, whose results may not be satisfactory. In this case the user returns to the design of the base set by changing the parameters of the design, and so on.

Furthermore, it is not possible to make data processing (i.e. simulations), with the concentrations actually used in the laboratory. In fact, PrimerExplorer works with fixed concentrations (oligonucleotide concentration of 0.1 μM, sodium ion concentration of 50 mM, magnesium ion concentration of 4 mM). This limitation prevents a reliable prediction of the melting temperature Tm.

The primer sets resulting from the data processing of PrimerExplorer therefore require a verification, carried through another software program able to provide "in silico" predictions of the melting temperature Tm. For this purpose it is considered effective to use Visual OMP TM (SantaLucia, and J. Hicks, D., "The thermodynamics of DNA structural motifs", Annu. Rev. Biophys. Biomol. Struct. 2004. 33: 415-40 ), primarily developed for the design of probes and primers to be used with the PCR technique.

Both PrimerExplorer and Visual OMP use, as the basis for calculation, a set of thermodynamic parameters published in the literature (SantaLucia, and J. Hicks, D., "The thermodynamics of DNA structural motifs", Annu. Rev. Biophys. Biomol. Struct . 2004. 33: 415-40). Visual OMP uses the algorithms of UNAFold (M. Zuker. Mfold web server for nucleic acid folding and hybridization prediction. Nucleic Acids Res. 31 (13), 3406-3415, 2003) for the prediction of intermolecular structures (hybridization) and intramolecular (folding).

Visual OMP uses the following formula for the calculation of Tm:

$$T_M = \Delta H^\circ \times 1000/(\Delta S^\circ + R \times \ln(C_T/x)) - 273.15$$

The Tm, as shown, changes according to the concentration (CT). The concentration is usually calculated considering a system with two states ("two-state model"), assuming that in the solution (or solvent or mixture of solvents in which the reaction occurs) there are only two species: the single strand of DNA and the primer, in the hybridized and not hybridized forms.

This represents an approximation since, in the solution, the present species are many (e.g. the other primers and their combinations). For this reason, Visual OMP optimizes the calculation of Tm considering the effective concentration of all the species in solution ("multi-state model"). The Tm calculated through this optimization is defined as Effective Tm.

Visual OMP allows the display of the melting curves. In these graphs the relationship between the concentration of the species and the temperature is represented. There are cases in which, with increasing temperature, the concentration ascend and then descend again. The consequence is that there are two points in which the target is hybridized to the 50% with the primer, and then two Effective Tm values (for example 12 ° C and 65 ° C).

A limitation of Visual OMP is that this system, in these cases does not provide the Effective Tm.

It was also verified that Visual OMP calculates the melting curves through an approximation. The thermodynamic parameters are evaluated at the assay temperature and used to evaluate the most stable structures. The ΔH and ΔS values are then used to calculate the ΔG at other temperatures (from 10 ° C to 100 ° C) through the following formula:

$$\Delta G_T^\circ = \Delta H^\circ - T \Delta S^\circ$$

This approach allows to quickly calculate the melting curves but assumes that the secondary structure of the dimer is always the same at different temperatures. This is true for the dimers formed by the primer and the complementary target DNA sequence, but not necessarily for other dimers which can hybridize to each other in a different way depending on the temperature.

Indeed, the primers can combine between forming primer dimers more or less stable. The formation of primer dimers, as demonstrated by experiments, generates unwanted amplification products. Is therefore important to be able to predict their formation and their stability considering the reaction conditions.

A more accurate calculation of melting curves should evaluate the stability of the secondary structures for each degree of the temperature scale.

The purpose of the present invention is to propose a design method of primers able to overcome these and other limitations and drawbacks of the known techniques mentioned above.

Such purpose is achieved with a computer-based method for the design of primers according to claim 1. The dependent claims describe preferred embodiments of the invention.

In accordance with claim 1, it is proposed a design method based on a computer for designing a set of primers to be used for an amplification reaction of a sequence of target nucleic acid, comprising the steps of:

a) providing a target nucleic acid sequence;
b) providing the conditions of the amplification reaction of the target nucleic acid sequence and design criteria of the primers, said design criteria comprising at least value ranges for the melting temperature, the ΔG limits of the hybridization between the end of a primer and the target nucleic acid sequence and hybridizations between the primers, and for the ΔG of the hairpin of a primer, the content in CG bases, and the distances between the primers composing the set;
c) selecting a candidate primer;
d) subjecting the primer to a hybridization algorithm to:

d1) predicting, from the multiple possible combinations between the candidate primer and the target nucleic acid sequence, the conformation of the most stable structure considering the values of ΔG, ΔH and ΔS;
d2) calculating from said values of ΔG, ΔH and ΔS the melting temperature between the candidate primer and the target nucleic acid sequence;

in the presence of at least one other previously selected candidate primer:

d3) predicting the conformation of the most stable of all the possible combinations of candidate primers considering the values of ΔG, ΔH and ΔS;
d4) calculating, from said values of ΔG, ΔH and ΔS, the concentration of all the structures present in the reaction environment;
d5) calculating, on the basis of said concentration value, the effective melting temperature between the candidate primer and the target sequence of nucleic acid;
e) subjecting the candidate primer to a folding algorithm to predict the conformation of the most stable structure of the hairpin of the candidate primer and calculate the ΔG, ΔH and ΔS values thereof;
f) comparing said values obtained from the hybridization and folding algorithms with the design criteria;
g) if the result of said comparison is acceptable, repeating steps b)-e) with another candidate primer until a set of primers is obtained, otherwise
h) changing the selection of the candidate primer.

The step of selecting a candidate primer provides for displaying on a computer screen the target nucleic acid sequence and selecting the candidate primer on said sequence.

After selecting a set of primers, a step i) of graphical representation of the dumbbell on the computer screen is provided for, prior to the confirmation of acceptance of the primer set.

The monomer dumbbell structure is predicted by means of the folding algorithm and represented graphically in order to permit the visualisation of any unexpected loops.

In a preferred embodiment, the design criteria comprise the length of the amplicon, and, at the end of the selection of a set of primers, a step i) of calculating the length of the amplicon of the selected primer set and a step 1) of comparing the range of desired amplicon length and the length of the amplicon of the primer set selected, are provided for.

In one embodiment, the method further comprises a step of assigning a score to a selected primer, on the basis of the proximity of the predicted parameters, corresponding to the design criteria, to corresponding target values.

In one embodiment, the method, further comprises a step of assigning a score to a set of primers selected, said score being calculated by means of the steps of:

- calculating a balancing score relative to the balancing of the melting temperature Tm of the pair of primers F3 and B3, F2 and B2, F1c and B1c, and possibly LF and LB, based on the proximity of the balance obtained from the predicted melting temperatures to a balance obtained from the design criteria,
- calculating a stability score relative to the stability ($\Delta G$) of the primer dimers using as predicted value the $\Delta G$ value of the most stable primer dimer, based on the proximity of the predicted value of $\Delta G$ to the corresponding design criterion,
- calculating the score of the primer set considering the contribution of the scores of the individual primers, of the balancing score and of the stability score.

In one embodiment, the method, further comprises the step of calculating an amplicon score relative to the length of the amplicon, defined as the distance between the end of the F2 portion and the end of the B2 portion.

In details, the steps d3) and d4) comprise the sub-steps of:

i) calculating all the possible combinations of dimers (primer dimers or target primers) and monomers (random coil or hairpin) using the correct thermodynamic parameters database depending on the experimental conditions of the reaction environment and hybridization and folding algorithms;
ii) predicting the most stable secondary structures (dimers and hairpins) and providing the $\Delta G$ of such structures;
iii) calculating the equilibrium constant K according to the formula: $\Delta G = -RT * \ln(k)$;
iv) calculating all the concentrations of the structures in the reaction environment, for example by means of an iterative method;
v) repeating the above step at predetermined intervals of the temperature scale.

In one embodiment, the step v) is performed by means of a repetition of the steps i)-iv).

In an alternative embodiment, the step v) is performed by re-calculating, for each temperature range, the $\Delta G$ using the formula $\Delta G° = \Delta H° - T\Delta S°$.

In a preferred embodiment, after step iv), a calculation of the percentage amounts relative to the concentrations of the structures in the reaction environment is performed.

More precisely, the actual melting temperature (actual Tm) of a structure is calculated as the temperature of the reaction environment at which the percentage amount of the concentration of the hybridized structure corresponds to 50%.

It is also object of the present invention, a computer program product, directly loadable in the memory of a computer, comprising software code portions suitable to implement the design method of a set of primers according to any of the above described embodiments, when the computer program product is run on the computer.

Further characteristics and advantages of the method according to the invention will anyhow be evident from the following description of its preferred embodiments, given for illustrative and not limiting purposes, with reference to the attached drawings, in which:

Figure 1 shows a diagram depicting the LAMP primers and their positioning on the target DNA sequence;
Figure 2 is a simplified flow chart showing the method of designing primer sets according to the invention, in a possible embodiment;
Figure 3 is a block diagram relating to the insertion of the reaction conditions and design criteria into the algorithm of calculation of the primer;
Figure 4 is a flow chart of the algorithm used to design a primer set in one embodiment referred to as "assisted mode";

Figure 5 is a flow chart of the steps of data processing of the algorithm used to design primer sets in an alternative embodiment, called "automatic mode";

Figure 6 is a flow chart of the selection of primer candidates in automatic mode;

Figure 6a is a flow chart of the algorithm of selection of primer sets according to the automatic mode, starting from the selected primer candidates with the algorithm of Figure 6;

Figure 7 is a flow chart relevant to the calculation of the concentration curves and the determination of the effective melting temperatures (Tm);

Figure 8 represents an example of a dumbbell with the two loops (forward and backward) having comparable size between them ("symmetric" dumbbell), and in which are also visible the backward inner primer (BIP) and the forward loop primer ( LF) with their binding positions;

Figure 8a represents another example of a dumbbell, in which is also visible the forward inner block of primers (FIP) with its position of binding;

Figure 9 represents an example of calculation of a target value for the calculation algorithms of a score to be assigned to a primer and to a primer set;

Figure 10 is a flow chart of the algorithm of calculation of the score of the individual primers;

Figure 11 is a flow chart of the algorithm of calculation of the score of the primer sets starting from the scores of the individual primers;

Figure 12 shows a graphical user interface relevant to the preparation phase of the basic system record, in particular the insertion of the design criteria;

Figure 13 shows a graphical interface of the algorithm to design a primer set with the assisted mode, in which are shown in particular the sequence of the target DNA, the selected primers, the dimers and the hairpins;

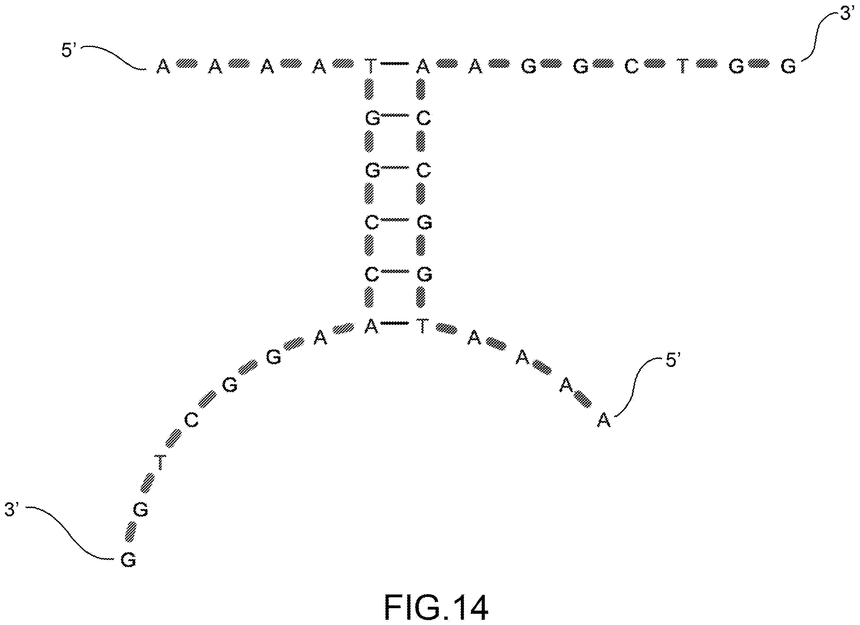

Figure 14 is a graphical representation of a secondary structure formed by a dimer;

Figure 15 is a graphical representation of the monomeric structure of a dumbbell as predicted by the algorithm of folding;

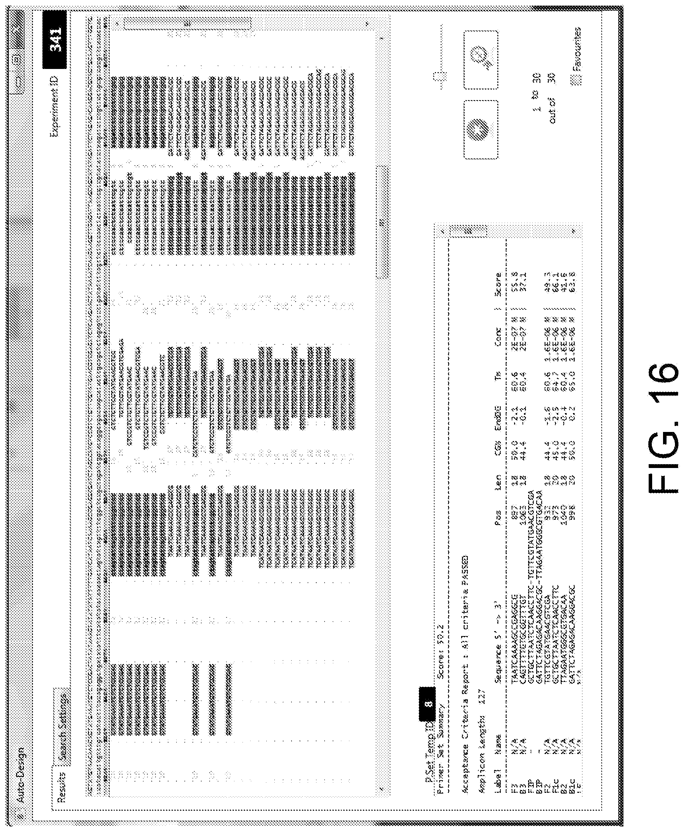

Figure 16 represents a visualization on screen of primer set candidates obtained with the automatic design mode;

Figure 17 is a graphical representation of an extensible dimer; and

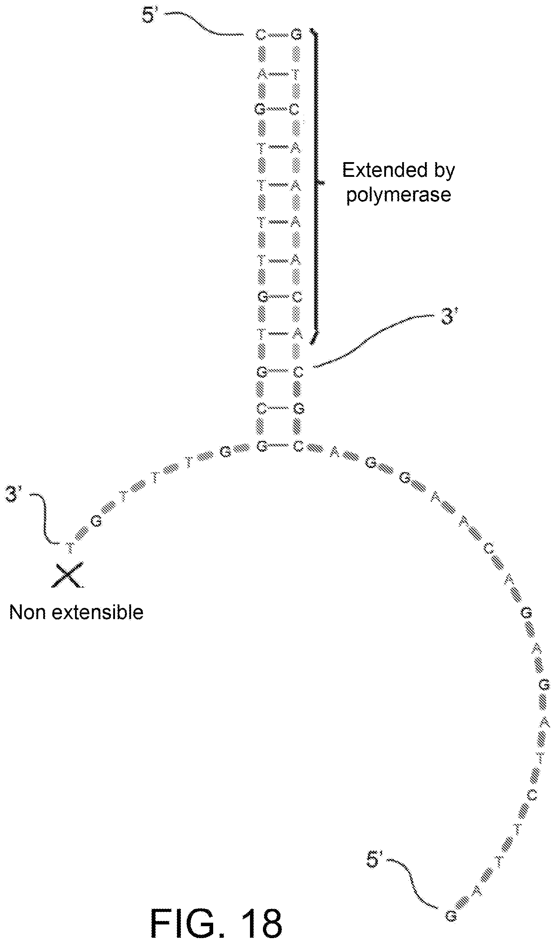

Figure 18 is a graphical representation of a dimer extended by the polymerase.

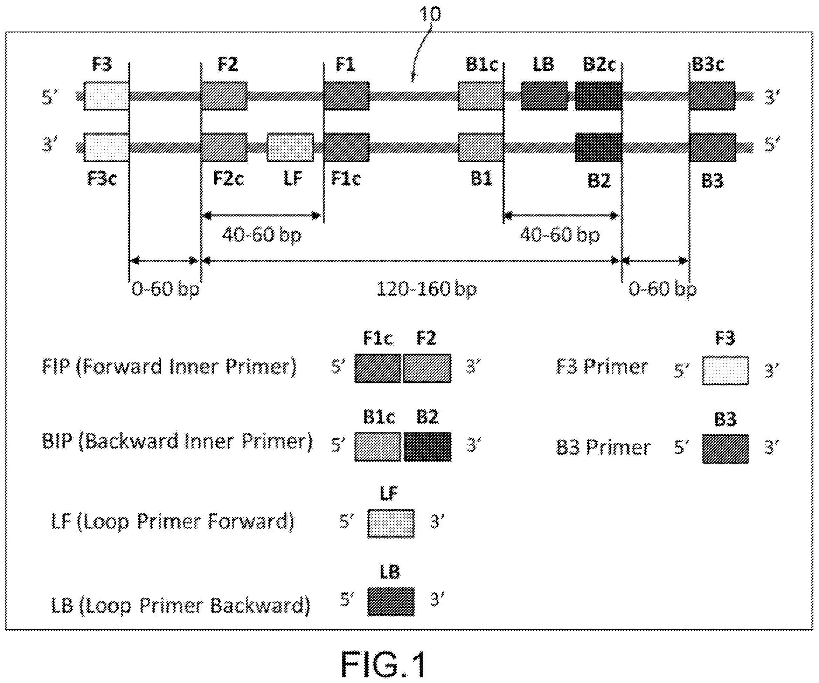

Figure 1 schematically represents a primer set positioned on a sequence of target DNA 10 that has to be amplified. It can be noted the set of basic primers, composed of F1c and F2 primer forming FIP forward inner primer, by B1c and B2 primers that form the BIP backward inner primer, and the outer primer F3 and B3, and forward loop primer LF, and backward loop primer LB, optionally present in addition to the basic set to act as catalysts agents of the amplification reaction.

The design method based on the computer according to the invention allows a LAMP researcher, through a series of graphical user interfaces, to design the LAMP primer sets starting from a DNA target sequence, considering the reaction conditions and design criteria.

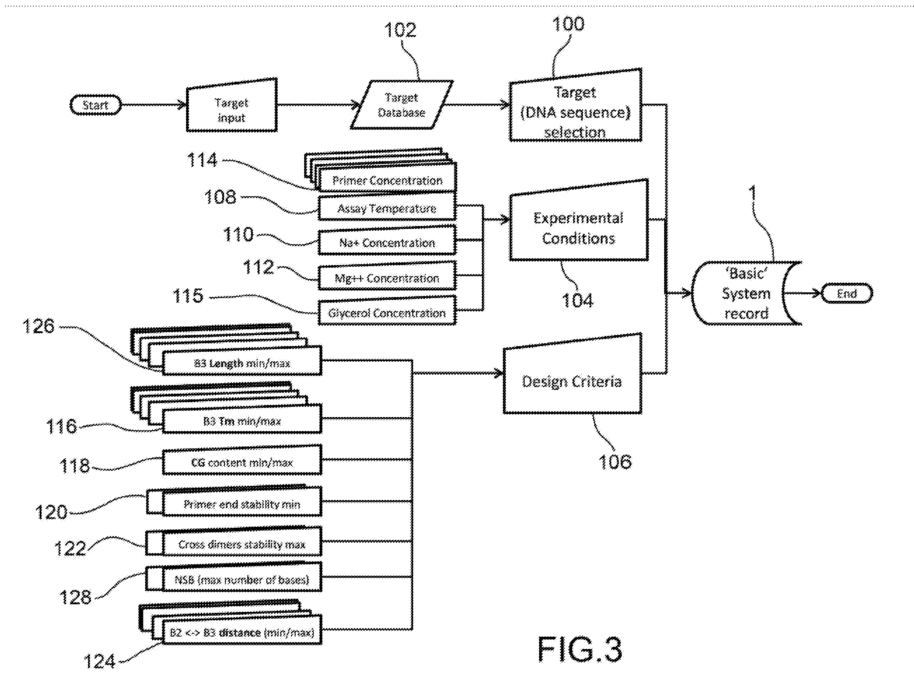

Block diagram of Figure 3, represents the preparatory steps of the design method according to the invention. In particular, steps are highlighted for selection and storage of a sequence of target DNA 100, for example starting from a database of target DNA sequences 102, which may be accessible by the program that implements the design method, and insertion and storage of the reaction conditions (104) and the design criteria (106).

In a preferred embodiment, the reaction conditions comprise: the assay temperature 108, the concentration of monovalent cations 110, the concentration of divalent cations 112, and the concentrations of the primers in solution 114. Optionally, the reaction conditions further comprise the percentage of glycerol 115.

It is noteworthy that different concentrations can be specified, in contrast for example to what happens in PrimerExplorer, wherein concentrations have a predetermined fixed value.

As for the design criteria, you can enter values for F3/B3, F2/B2, F1c/B1c and LF/LB pairs.

The design criteria include, for the above pairs: the melting temperature Tm 116 (°C, minimum and maximum value), the content in CG bases 118 (percentage, minimum and maximum value), the stability threshold of the 3' and 5' ends (120) ($\Delta$G, minimum value), the stability threshold relevant to the primer dimers (122) ($\Delta$G, maximum value), the set of distances separating the various primers (124) (number of bases, minimum and maximum values).

As mentioned above, the primers can be combined with each other giving more or less stable primer dimers. The formation of primer dimers, seen experimentally, generates unwanted amplification products.

Additional design criteria may be, for the above pairs, the length 126 (number of bases, minimum and maximum value), and the number (threshold) of bases of non-specific bonds (Not Specific Binding) NSB 128.

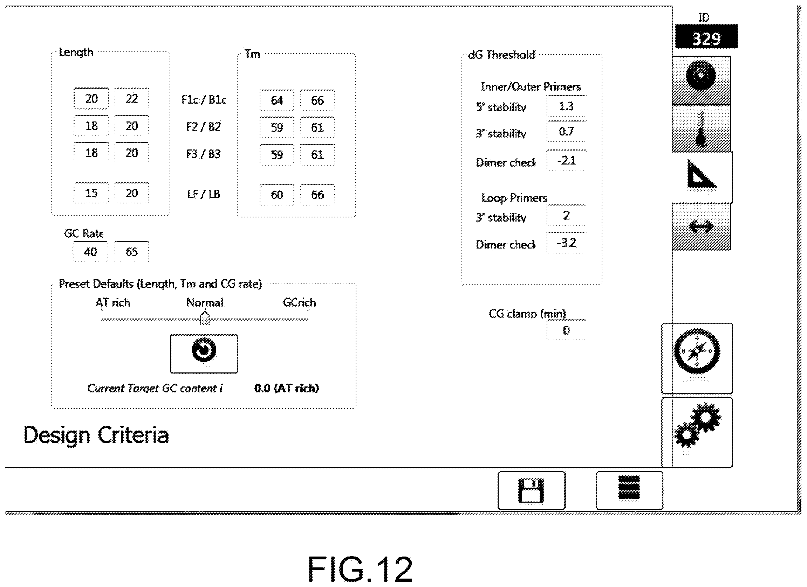

A sample of graphical user interface for the insertion of the design criteria is shown in Figure 12.

The sequence of the selected target DNA 100, the reaction conditions 104 and the design criteria 106 form a 'basic' system record 1 for the algorithm that implements the design method according to the invention.

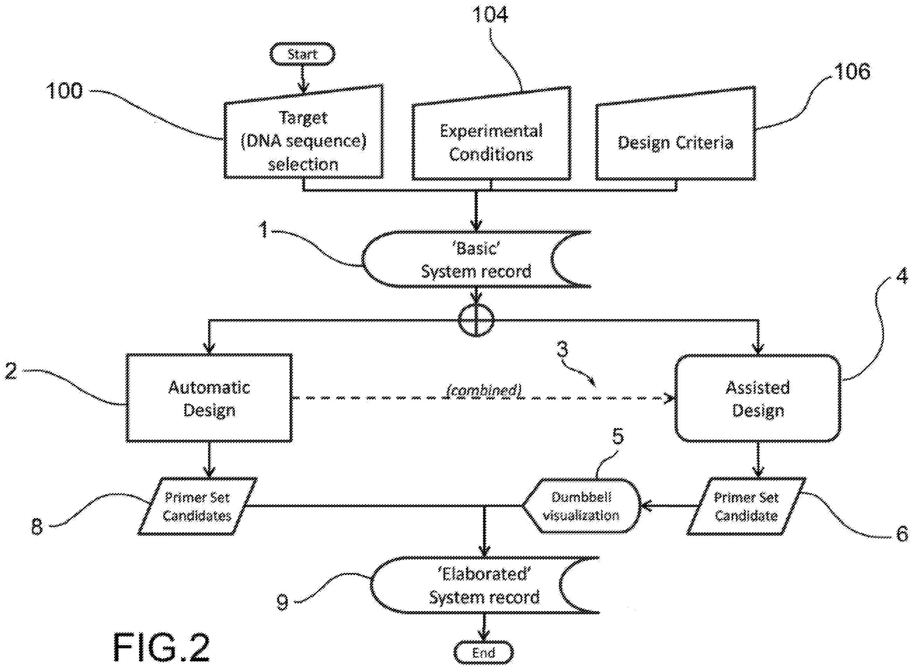

As represented in the block diagram of Figure 2, once the sequence of the target DNA 100, the reaction conditions 104 and the design criteria 106 have been entered and stored in the computer, the user can decide whether to perform an assisted design (4 ) of a primer set (6) or an automatic design (2) of primer sets (8).

In a preferred embodiment, the primer set candidate 6 obtained with the assisted mode is represented graphically through the dumbbell display 5 for a final assessment by the researcher, as will be described later.

In both design modes, the primer set candidates, which have possibly passed the evaluation based on the graphic representation, are stored in an 'elaborated' system record 9 of the design algorithm.

As it will be described in more detail below, in the assisted mode the user selects sequences (primers) directly on the target sequence. The design algorithm performs a calculation of the thermodynamic parameters relevant to the sequence and the secondary structures formed by it with the other sequences in solution. Conditional formatting allows the visualization of possible discrepancies with the design criteria. For example, the values that meet the acceptance criteria are displayed in green, the values that do not respect them are displayed in red.

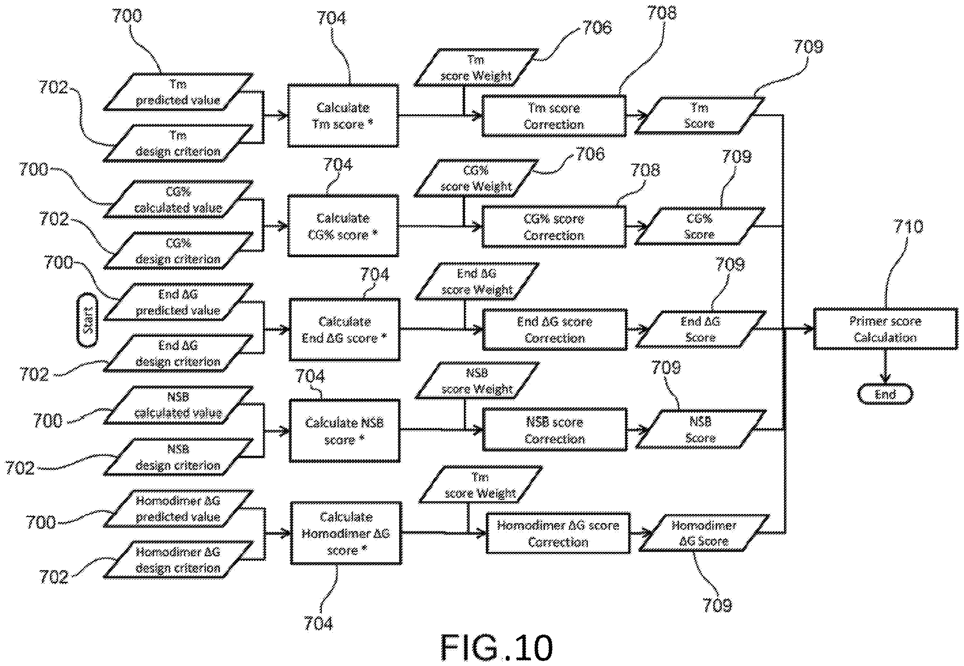

In the automatic mode, the design algorithm performs a scan along the selected target sequence, on the basis of the design criteria, identifying the possible primers and assigning them a score (for example, based on proximity to the value or range of values specified in the design criteria, as will be exemplified below with reference to Figure 10. The primers with a better score (primer "candidates") are then evaluated, in combination, by means of the algorithm ahead described, in order to constitute the best (to score) "in silico" primer sets.

In one embodiment that can be defined as "combined mode" (3), the sets of primers obtained with the automatic mode (2) can be transferred to the assisted mode (4), in order to be changed according to the user's preferences.

It is worth noting that that the design criteria represent a barrier in the automatic mode, or the sequences that do not comply with the criteria are excluded. In the assisted mode, however, such sequences are eligible, and are distinguished by sequences that meet the design criteria, for example by means of special symbols.

After setting the target, the reaction conditions and the design criteria you can switch to the design through the assisted mode, automatic or with the combination of both.

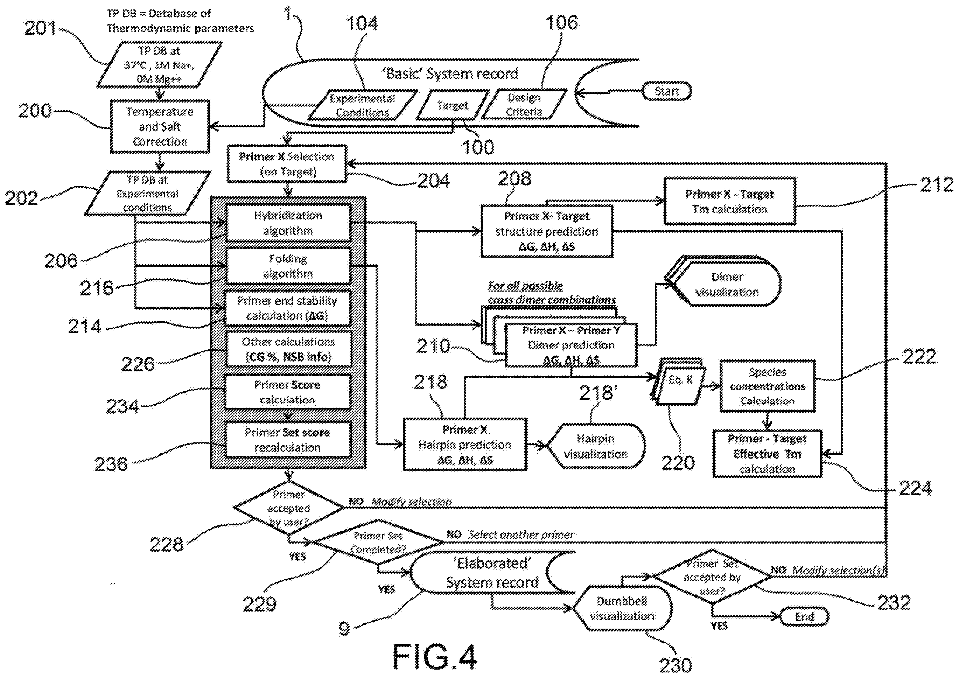

It will now be described, with reference to flow chart of Figure 4, the algorithm to design a primer set according to the assisted mode.

In the assisted mode (Figure 4) the user, starts from a 'basic' system record storing: the target DNA sequence 100, the reaction conditions 104 and the design criteria 106. Preferably, the design method allows also the specifications of the values of the solution conditions (step 200), such as temperature and salt concentrations, in such a way to perform a possible automatic update of the thermodynamic parameters database 202, compared to the standard reaction conditions (201), for example in which the temperature of the solution is 37 °C and the concentrations of monovalent and divalent ions are respectively of 1M and 0M.

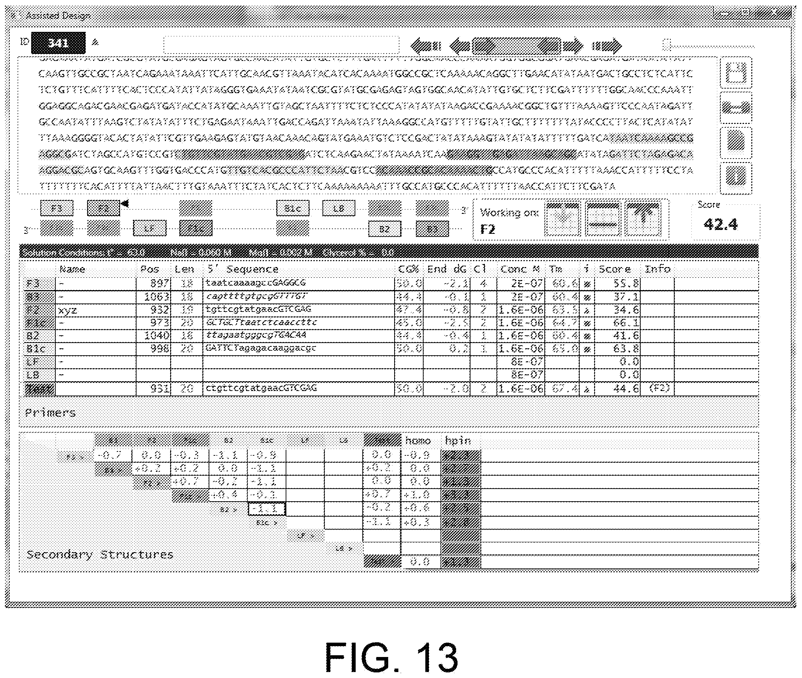

Then the program allows the user, for example through a graphical interface of the type illustrated in Figure 13, to select the sequences (primers) directly on the text string of the target sequence (step 204). The selection of the primer (sequence of nucleotides) is then sent to the algorithm of hybridization (step 206), which predicts the most stable structures of dimers through a calculation of the thermodynamic parameters $\Delta G$, $\Delta H$, $\Delta S$, both in the case primer-target (step 208) and in the case of primer dimers (step 210), for all possible combinations of dimers.

More in detail, once the thermodynamic parameter database (201) is corrected (202) according to reaction conditions (200), using the $\Delta H$ and the $\Delta S$ at 37 °C gives $\Delta G_T^\circ = \Delta H^\circ - T\Delta S^\circ$

that are the values of $\Delta G$ of the possible Watson-Crick pairs, of the mismatches, of the loops (2004 SantaLucia Hicks -THE THERMODYNAMICS OF DNA STRUCTURALMOTIFS, page 419, and tables 1,2,3 etc ...). At this point, the algorithm compiles a matrix of $\Delta G$ values obtained by evaluating all possible combinations.

Subsequently, the algorithm identifies, in this matrix, the point with the lowest $\Delta G$ and from this, through a dedicated function, is able to identify the structure that has such $\Delta G$. As soon as a part of the structure is identified, the algorithm adds the $\Delta H$ (relevant to the part of the structure) to the $\Delta H$ calculated up to there (structure $\Delta H$).

In other words, the algorithm, starting from the corrected thermodynamic parameters database, uses the calculation of $\Delta G$ to identify, among the many possible combinations, the most stable conformation and, consequently, its values of $\Delta G$, $\Delta H$ and $\Delta S$.

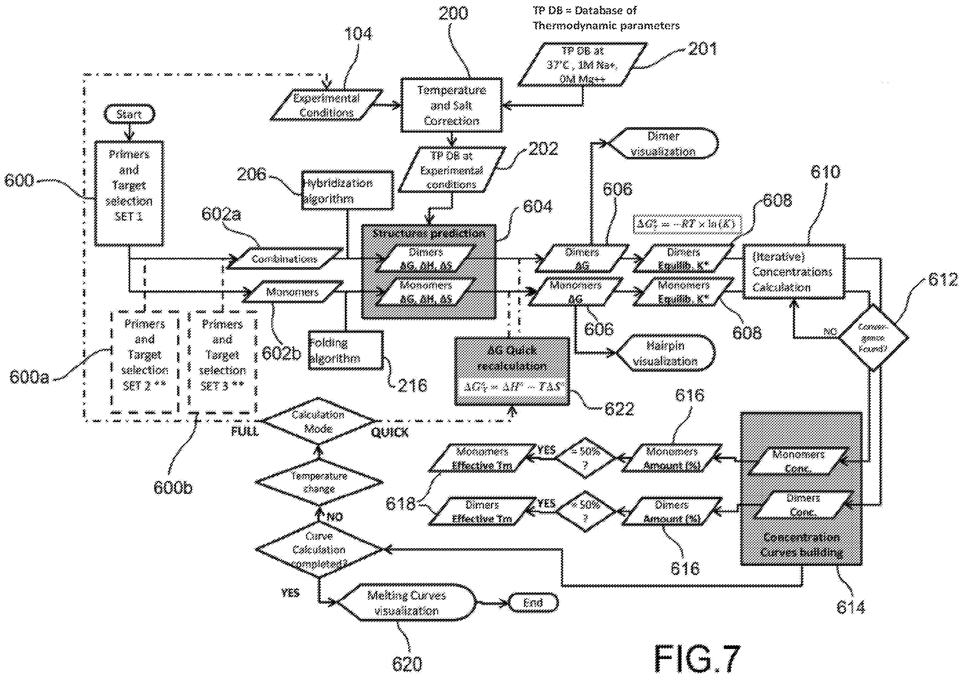

The $\Delta G$ of primer-target dimer allows the calculation of the melting temperature Tm of the primer (step 212). The selection of the primer is also used by the program to calculate the stability ($\Delta G$) of the primer ends (step 214) and to predict, by applying the folding algorithm (step 216)), and display, the more stable hairpin (steps 218, 218'). The $\Delta G$ of the aforementioned structures (dimers and hairpins) allow the calculation of the equilibrium constant K (step 220) and consequently of the concentrations of all species (structures) in solution (step 222). These concentrations allow the calculation of the effective Tm primer-target (step 224), as will be described below in more detail with reference to Figure 7 flow chart.

In one embodiment, the algorithm provides in real-time the above-mentioned thermodynamic parameters along with the GC content percent and the possible non-specific binding points (step 226).

Conditional formatting allows the clear visualization of possible discrepancies between the predicted values and the design criteria, both for the individual primers, either for their combinations (dimers).

If the selection results are not accepted by the user, it is sufficient to change the selection of the primer (step 228).

The algorithm is repeated, starting from step 204 of selection of the primer on the target DNA sequence, up to complete the selection of the primer set (step 229).

In a preferred embodiment, once the entire set of primers is selected, the assisted design algorithm graphically displays the key structure of the LAMP amplification mechanism, which is the dumbbell (step 230) .

If the dumbbell is not accepted by the user, it is sufficient to change the selection of the primer/primers to obtain a new key structure (step 232).

In a preferred embodiment, after the prediction of the values of the parameters corresponding to the design criteria, the algorithm assigns a score to each primer selected based on the proximity of these parameters to corresponding target values (step 234). An example of the calculation of the target values and an example of the score calculation are described later with reference to Figures 19 and 20.

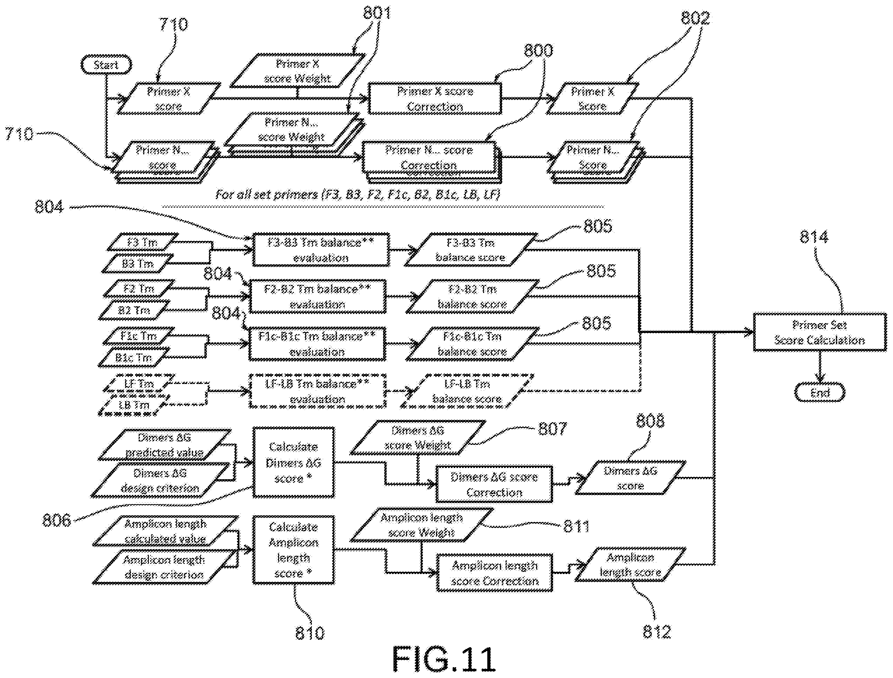

Furthermore, in one embodiment, the algorithm assigns a score also to the selected primer set (step 326), according to the algorithm described later with reference to Figure 11.

When the design includes the use of the loop primers (one or both), these are also depicted in order to display their specific binding area.

The dumbbell structure, generated from the annealing and the folding of the FIP and BIP primers on F1 and B1 sequences of the target, allows the further annealing at specific sequences which are targets of inner primers as well as of the loop primers and the subsequent polymerization of the DNA. This cycle of amplifications can be initiated either from the forward side (or directed) that from the backward side (or reverse) of the dumbbell. Indeed two types of dumbbell will be present in solution:

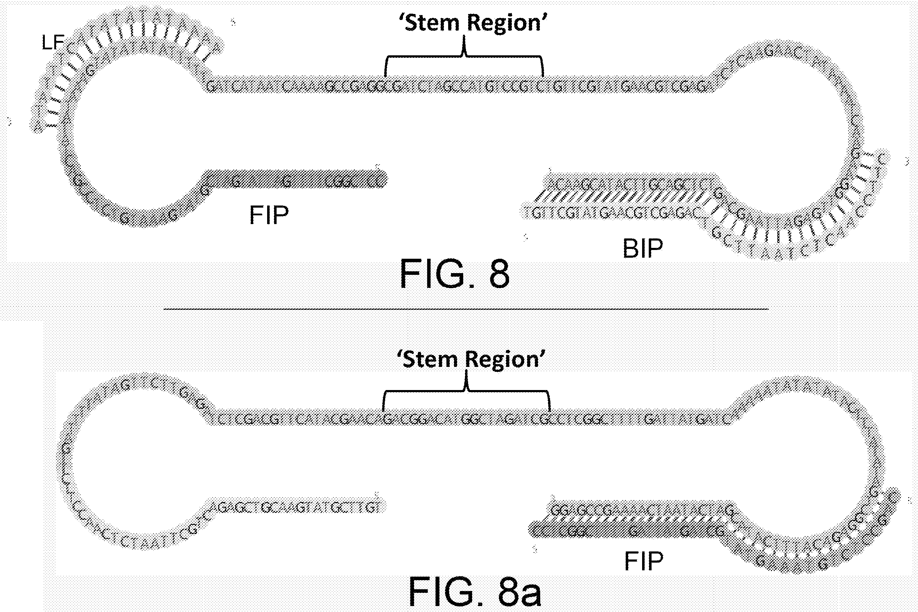

(i) a dumbbell on which the LF primer (when present) and the BIP primer (Figure 8) will anneal: the LF primer will bind on the loop generated by the folding of the FIP primer, while the BIP primer will bind at the complementary target sequence at the level of the second loop;

(ii) a dumbbell on which the LB primer (when present) and the FIP primer (Figure 8a) will anneal: the LB primer will bind on the loop generated by the folding of the BIP primer, while the FIP primer will bind at the complementary target sequence at the level of the second loop.

The display of the dumbbell is of fundamental importance in the design phase of the primers, since it allows to evaluate the size of the dumbbell and of the loops, the symmetry (or asymmetry) of the dumbbell, the optimal position of binding of the loop primers and the optimal position of primers called "stem primers" which are the primers selected in the area ("stem region") between F1c and B1c of the target, which will be the central part of the dumbbell (Figure 8).

The size of the dumbbell is linked to the length of the amplicon, that is, the region amplified by the LAMP method. Since there is a direct correlation between the amplicon length and the speed of the amplification (i.e. small amplicons are generally amplified faster than larger amplicons), the assessment of the size of the dumbbell is fundamental to compare the sets of primers and identify the potentially most promising primer sets according to the design needs. For example, in the case where the purpose is to design a very fast and sensitive primer set capable of detecting a low quantity of target copies a small amplicon will be preferred; in the case where it is intended to design a set of primers for a housekeeping gene that acts as a weak internal control of the amplification reaction, a slightly larger amplicon will be preferred.

In addition, an analysis of the size of the loop is required to assess the possibility to design the loop primers in such region. Let's assume the case it is desired to design two primer sets for two different fusion transcripts which have a common backward sequence and two different forward sequences, wherein design two forward loop primers labeled with different fluorophores for the discrimination of the two transcripts. One of the applicable design strategies in this case would be to draw a dumbbell that present a forward loop of a size slightly larger than the backward loop, so as to ensure further design possibilities and therefore have a greater probability of generating loop primers which have optimal quenching properties.

Another important aspect of the dumbbell visualization is represented by the possibility to view the position of the primers on the dumbbell: this is useful for both the loop primers, designed on the loops of the dumbbell, both for "stem primer," which are drawn in the single-stranded portion between the two loops. Their visualization with the dumbbell allows better accuracy to assess the region in which they should be placed, also by comparison with any prior designs.

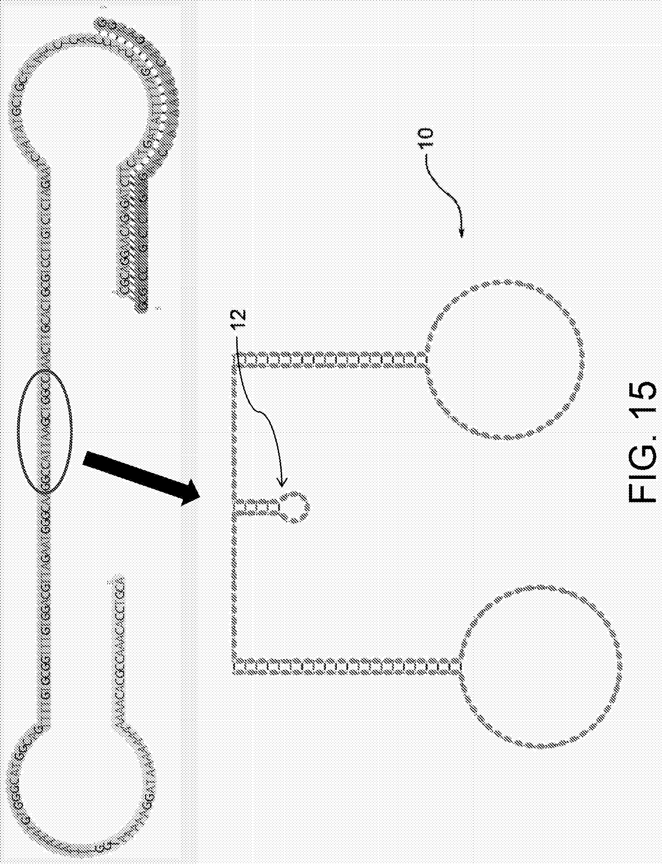

Considering that the structure of the dumbbell is nothing more than a monomeric structure, that is a single strand, which forms a particular type of hairpin with two loops, in a preferred embodiment, such a structure can be generated by the algorithm of folding. In this way, in graphic representation 10 of the dumbbell, any unexpected loop 12 can be highlighted (Figure 15). If present, these loops are immediately detected by the user, which may decide whether to make another selection of the primers.

Therefore, the design method according to the invention can apply the folding algorithm both to predict the more stable hairpin, and to generate the structure of the dumbbell.

According to a further aspect of the invention, the design method allows the selection in the graphic representation 10 of the dumbbell, a new loop primer sequence in a different position compared to the one originally represented, and the subsequent recalculation of the values of the parameters corresponding to the design criteria.

This new position of the selection of the loop primer results in a modification of the bases which make up the loop primer sequence (preserving the complementarity of the bases to the target sequence).

In this way, the researcher can immediately verify the impact of the new sequence on the previously calculated parameter values corresponding to the design criteria.

For example, the researcher may want to evaluate the loop primer sequences that end with specific bases, for example CC on the 5' end, at the same time taking into account the geometric position of the loop primer sequence along the relevant loop of the dumbbell structure.

It is important to note that, in this assisted mode of the design algorithm, the sequences (primers) are selected directly on the text string of the target sequence manually by the designer. Unlike other algorithms of automatic design, a primer is therefore not rejected from the start only because it does not meet certain design criteria. For example, once the sequences that meet the design requirements have been identified, the designer could work on sequences close to those selected to see how certain sequences interact. For example, primers that do not fully meet the design criteria may give more stable bonds than primers that meet the criteria.

Also noteworthy is the fact that to calculate the thermodynamic parameters at the assay temperature allows to determine more precisely which dimers are formed at a given temperature. For example, more stable dimers which are formed at 37 °C likely do not match those that are formed at the assay temperature (e.g. 60 °C).

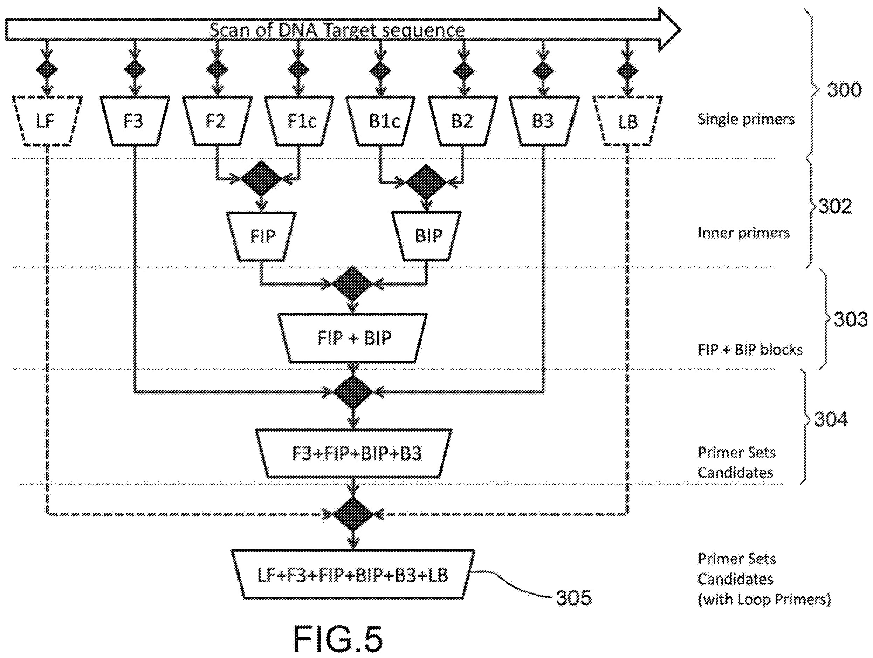

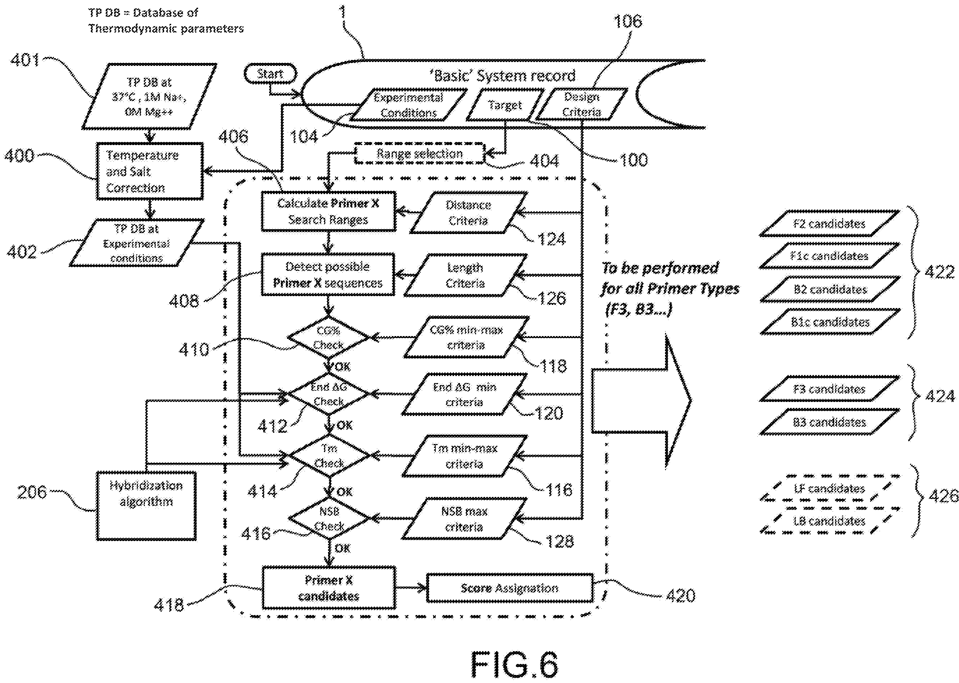

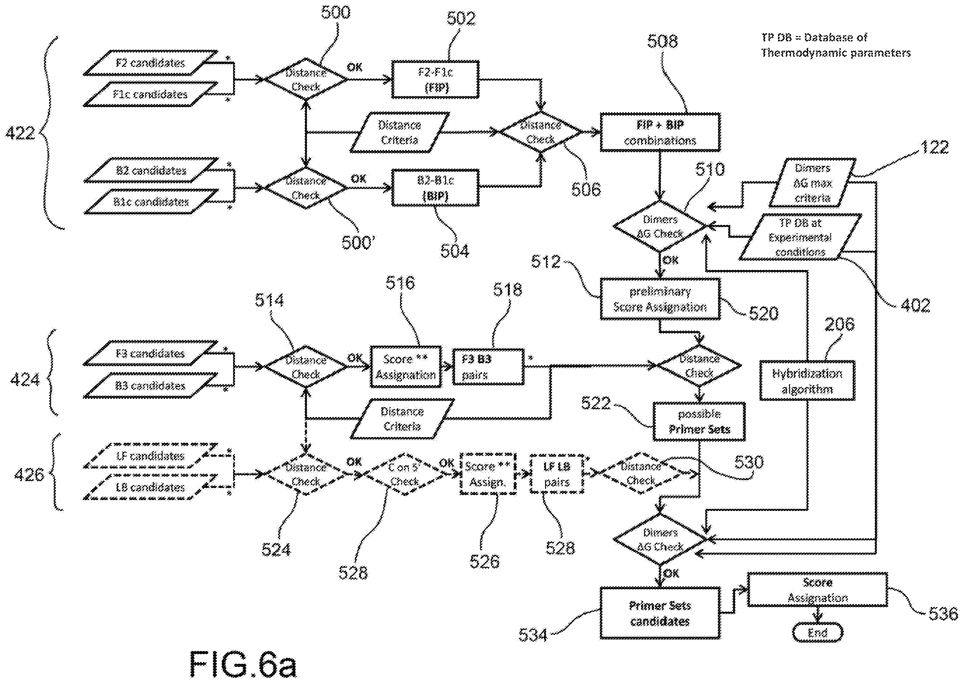

It will now be illustrated, with reference to the flow charts of Figures 5, 6 and 6a, the design algorithm of primer sets with the automatic mode.

Figure 5 represents, briefly, the logic used by the algorithm to identify primer set candidates.

As before, a 'basic' system record is provided where the target sequence, the reaction conditions and the design criteria are registered.

In a first step (300), the program scans the target sequence to identify all possible F3, which are all the sequences that meet the design criteria related to F3. The scan is then carried out also for other types of primers (F2, F1c, etc.) including, where appropriate, the loop primers (optional). At the end of the first phase, all possible primers, broken down by type, are identified.

In a second step (302), the algorithm identifies the possible inner primer (FIP and BIP) considering combinations F1c + F2 and B2 + B1c that meet the design criteria (for example, the distance criterion). These criteria are also applied in a third step (303), where the system selects the possible combinations FIP + BIP, and in a fourth step (304), where the FIP + BIP combinations are combined with the outer primers (F3 and B3) to obtain the primer set candidates.

If loop primers are expected, the algorithm, always according to the distance criteria, combines the primer set candidates to the loop primers (step 305).

Figure 6 and Figure 6a describe in detail the design algorithm of the automatic mode.

As in the assisted mode (Figure 6) the user, starts from an 'basic' system record storing: the target DNA sequence 100, the reaction conditions 104 and the design criteria 106. Preferably, the design method allow also the specifications of the values of the solution conditions (step 400), such as temperature and salt concentrations, in such a way to perform a possible automatic update of the thermodynamic parameters database 402, compared to the standard reaction conditions (401), for example in which the temperature of the solution is 37 °C and the concentrations of monovalent and divalent ions are respectively of 1M and 0M.

The user can optionally select a part of the target (404). On the whole target or on a part selected by the user, the algorithm calculates the search range (406) for the individual primers, for example starting from the F3 primer, according to the design criteria relating to the distances (124, see Figure 3).

All the sequences that meet the criterion of length 126 relevant to the primer in question (step 408) are then identified, along this interval.

The sequences are then subjected to subsequent checks to verify if they specific design criteria. The sequences that do not pass the checks are discarded.

The first check (step 410) relates to the CG nucleotide content percentage 118. The next checks (step 412, 414) verify the stability 120 ($\Delta$G) of the ends and the melting temperature Tm 116, both calculated using the hybridization algorithm (206).

The final check (step 416) verifies a parameter related to the possibility of non-specific bond 128 (NSB Non Specific Binding).

If there are areas, on the target sequence, where the primer sequence can hybridize with a number of nucleotides greater than design criteria limit (and different from the specific point of binding), the primer sequence is discarded. At the end of these checks a series of candidate primer candidates (418) is obtained to which is assigned a score (step 420), for example, the higher the more its parameters are corresponding to the design criteria 106.

The selection process is repeated in order to obtain all the necessary primers to form a set, i.e. all inner primers 422, outer primers 424 and optional loop primers 426.

Figure 6a describes in detail the process by which the design algorithm identifies the primer sets from the individual primer candidates 422, 424, 426.

A first step 500 includes the identification of the possible FIP 502 starting from the best (by score) F2 and F1c candidates, considering the distance design criteria 124.

Similarly the possible BIP 504 starting from the best (by score) B2 and B1c candidates are identified (step 500') .

Then the possible combinations FIP + BIP 508 are selected (step 506), always considering the distance design criteria 124.

Such combinations are subjected to a check relevant to the stability of primer dimers (step 510) that uses the relevant design criteria 122, the thermodynamic parameters database 402 corrected by using the reaction conditions 400 and the hybridization algorithm 206.

A preliminary score (step 512) is assigned to the FIP + BIP combinations that are not discarded (i.e. the ΔG of the most stable dimer is greater than the limit specified in the design criteria).

The algorithm evaluates the best (by score) F3 and B3 candidates (outer primers 424) and compares them (step 514) with the distance design criteria 124, as it did for inner primers 422. A score is then assigned (step 516) to the selected F3 and B3 primer pairs on the basis of the balance (proximity) of the melting temperatures Tm of the two primers. Best F3 B3 pairs 518 are matched to the best combinations FIP + BIP (step 520) according to the distance design criteria in order to get the possible primer sets 522.

In one embodiment, the user can also indicate the amount of F3 B3 pairs to be matched to any combination FIP + BIP, in order to obtain additional primer set variants.

If the design includes loop primers 426 (both or one of the two), the algorithm evaluates the best (by score) LF and/or LB candidates and compares them with the distance design criteria 124 (step 524). A score is then assigned to the LF and LB primer pairs thus selected (step 526) based on the balance (proximity) of the Tm of the two primers (19).

In one embodiment, the algorithm optionally applies a particular design criterion for the primers loop (step 528) which allows to select only the loop primers whose end at the 5' end with a C nucleotide (i.e. those that allow the 'quenching').

Best LF LB pairs 528 are combined with primer sets 522 previously selected in compliance with the distance design criteria (step 530).

The user can also indicate the amount of LF LB pairs to be combined with each set, so as to obtain further variants.

The resulting primer sets 522 with, or without, primer loops, are compared to the acceptance criterion relevant to the stability of the primer dimers 122 (step 532) .

The sets that meet this criterion (i.e. the ΔG of the most stable dimer is above the limit indicated in the design criteria) result to be the primer set candidates 534 to which is assigned a score (step 536).

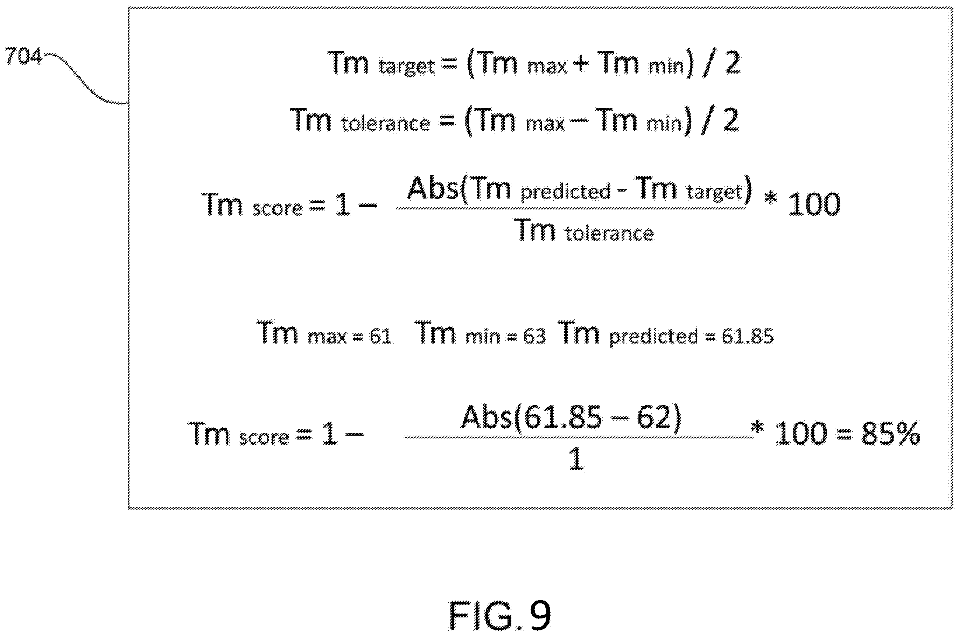

Will now be described, with reference to Figures 9, 10 and 11, an example of calculation of the score to be assigned to a primer (Figure 10) and a primer set (Figure 11), applicable both to the algorithm relevant to the assisted design and to the one relevant to the automatic design.

In a preferred embodiment, the score is directly proportional to the proximity to a target value of the value predicted by the design algorithm of a parameter associated to a primer. An example of the target value calculation is shown in Figure 9. The target for the melting temperature Tm is considered as the interval center of the design criterion (i.e. the mean value between Tm max and Tm min), the tolerance is the same interval divided by two. The example illustrates how a predicted melting temperature Tm (61.85) rather close to the target value (62) produces a rather high score (850).

In the calculation of the score to be assigned to a primer (Figure 10), the calculation algorithm compares the predicted value 700 of a parameter associated to a primer, for example the value of the melting temperature Tm, with its design criterion 702 and calculates the score (step 704).

This score can be corrected according to a weight 706 configurable in the program (step 708) (e.g. it is possible to give more weight to the melting temperature Tm than to the CG content percentage).

The assignation of the score is then carried out, with the mechanism described above, also for the predicted values of the stability (ΔG) of the ends, and of homodimers and for simply calculated values of the CG percentage and the greater number of bases of non-specific binding ( "NSB", Not Specific Binding).

At the end, the algorithm calculates the primer score (step 710) considering the contribution 709 of the parameters scores.

The assignation of the score to a primer set (Figure 11) takes place with the following calculation algorithm.

In one embodiment, in a first step 800, the calculation algorithm adjusts the scores 710 of the individual primers with a weight 801 assigned to them. Weighted scores 802 of individual primers are then obtained.

In a second step 804, the calculation algorithm, evaluates the balance of the melting temperature Tm of a series of primer pairs (F3 and B3, F2 and B2, F1c and B1c, and possibly LF and LB), and assigns a score to such balance (step 805). The greater the proximity between the values of the two pair primers the higher the score.

In a third step 806, the calculation algorithm calculates the stability score (for example as in Figure 9) relevant to the stability ($\Delta G$) of the primer dimers using the predicted $\Delta G$ value of the most stable primer dimer.

Also in this case, in one embodiment, the calculation algorithm adjust the stability scores of the dimers considering a weight 807 assigned to them. You then get stability scores weighted 808. Weighted stability scores of the dimers 808 are then obtained.

In one embodiment, the calculation algorithm also calculates (step 810), an amplicon score relevant to the amplicon length, i.e. the distance between the ends of the F2 portion, and the ends of the B2 portion. Also in this case the calculation is made in a manner similar to the one described in Figure 9.

In one embodiment, the calculation algorithm adjust the amplicon score considering an amplicon length weight 811. Weighted amplicon length scores 812 are then obtained.

At the end, the algorithm calculates the primer set score considering the contribution of the scores, possibly weighted, of the individual primers, of the balance, of the stability of the dimers and possibly the amplicon length (step 814).

The optimal length of a LAMP amplicon should be between 120 and 160 bases. Although it is not possible to predict the performance of a set considering only the distance of the primers that comprise it, experimental evidence shows that sets that form small dumbbells are generally faster than the sets that form larger dumbbells. The attribution of a score to the amplicon length could therefore help to discriminate between potentially faster sets among the ones designed by the software program.

In one embodiment, the design algorithm allows to select, as valid, only the loop primers that end with a specific sequence (for example CC on the 5' end).

Among the analytical determination methods based on the transfer of fluorescence energy, the 'quenching' in fluorescence induced by hybridization it has been developed in LAMP applications, in particular through the principle of 'quenching' with guanine (Zerilli et al. 2010. Clin Chem 56: 1287-96). In this approach the fluorescence emitted by a LAMP loop primer marked on the 5' end progressively extinguishes (=' quenching ') following the hybridization with a complementary target sequence containing a guanine. The intensity of the extinction effect depends on the number and on the positions of adjacent G bases on the complementary target sequence. When the target sequences accumulated in a real-time LAMP assay, quantitative measurement of the amplification of the nucleic acid can be obtained by monitoring the amount of fluorescence extinct as a result of the amalgamation of the labeled primer loop (dye-labeled ) in the amplification products. This strategy therefore depends on the specific nucleotide sequence of the nucleic acid target, in particular on the presence and/or positioning of guanine bases within that sequence.

According to one embodiment, it is also possible, unlike for example in PrimerExplorer, to set different design criteria for the two sides (forward, or direct, and backward, or the reverse).

One of the key factors in the LAMP primer design is represented by the correct distance between the primers. In particular, the primers should be designed so that:

- the distance between the end of the F2 portion, and the end of the B2 portion (region amplified by the LAMP method and corresponding to the amplicon length) is preferably comprised between 120 and 160 bases;
- the distance between the 5' end of the F2 portion, and the 5' end of the F1 portion (i.e., the region that forms the loop, where the loop primers hybridize) is preferably comprised between 40 and 60 bases;
- the distance between F2 and F3 is preferably comprised between 0 and 60 bases (Figure 1).

Taking into account this series of restrictive rules, the PrimerExplorer software program allows to design primers in a rather limited manner, generating only dumbbells in which the two loops (forward and backward) have comparable dimensions (Figure 8).

However, during the step of primer design, there is often the need to have available a larger number of possible alternatives to the standard design format, generating dumbbells of various sizes.

For example, the design of a primer on a specific chromosomal translocation, or on a specific genetic mutation requires that the primers are located in a specific and well delimited area of the target, so that the amplification of the target sequence may take place, and then genetic diagnosis can be performed. This can result in several difficulties, in the case in which the genomic region of interest is particularly rich in GC or, on the contrary, in the AT, or in the case in which it contains palindromic or homologous sequences to other genomic regions, where it is preferable to avoid the design. In these cases it may help to select on the target the forward primers or the backward primers respectively further upstream (towards the left) or further downstream (to the right) of the point where the translocation / mutation is localized, generating a dumbbell with a conformation different from the one allowed by PrimerExplorer.

Therefore, in one embodiment, the design algorithm allows different design criteria for the loop primers forward and backward.

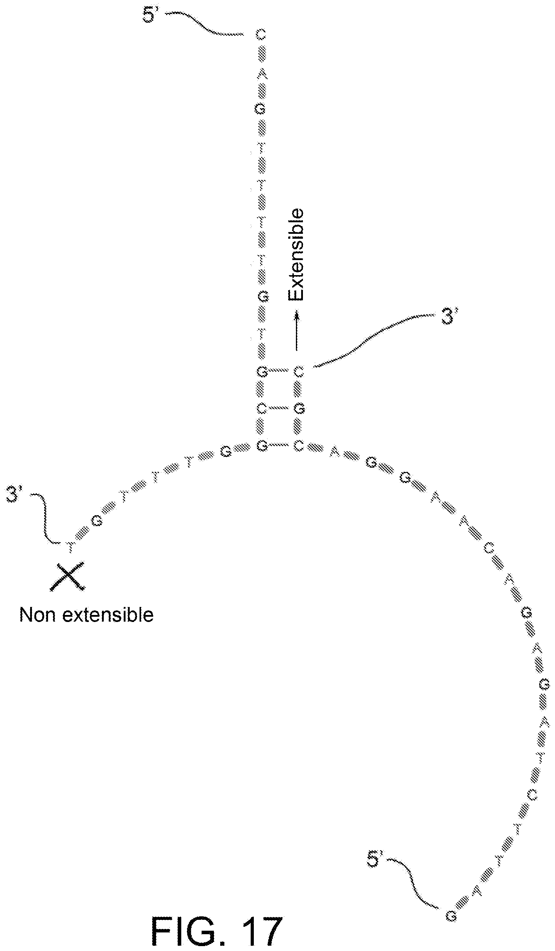

In one embodiment, the algorithm allows to evaluate which secondary structures (of primer dimers) are extensible on the 3' end (Figure 17), that is having parts on which the polymerase can easily take action by adding the nucleotides. This is not a desired characteristic and it is important to detect it. The algorithm also provides the measure of the extensibility, that is how many nucleotides can be added by polymerase.

As regards the calculation of the thermodynamic parameters, in a preferred embodiment, the effective Tm is calculated considering the concentrations of all the species in solution.

Unlike what happens for example in Visual OMP, if two values of effective Tm are possible, due to the trend of the concentration as a function of the temperature (melting curve), the design algorithm according to the invention provides the most value significant, that is the one close to the assay temperature.

With reference to Figure 7, unlike Visual OMP, the algorithm allows to calculate the melting curves by making the most stable structures for each degree of the temperature scale.

Figure 7 shows the detail of how the design algorithm allows to calculate and display all the secondary structures relevant to one or more sets, providing parameters such as $\Delta G$, Tm, effective Tm, concentration and percentage in solution.

In one embodiment, the user can select a recorded record (step 600), which contains information about the primers and the target. In addition to that record, others can be selected (600a, 600b).

The algorithm processes (step 604) all the possible combinations of dimers of primers or of primer-target 602a and monomers, random coil or hairpins 602B using for example the corrected thermodynamic parameters database 202 (step 200), according to the specified experimental conditions 104 (assay temperature and salt concentrations), compared to the standard reaction conditions (201), for example in which the temperature of the solution is 37 °C and the concentrations of monovalent and divalent ions are respectively of 1M and 0M.

The algorithm applies the algorithms of hybridization 206 of folding 216 to the possible combinations of dimers of primers or of primer-target 602a and monomers, random coil or hairpins 602B.

The algorithm consequently predicts the more stable secondary structures (dimers and hairpins) and provides the $\Delta G$ (step 606).

Starting from the $\Delta G$ is possible to calculate the K equilibrium constant (step 608) according to the formula:

$$\Delta G_T^\circ = -RT \times \ln(K)$$

Considering that all the equilibrium constants of the species (or structures) in solution are then available, it is possible, for example, through an iterative method, to calculate all the concentrations of the species (or structures) in solution (step 610). The iteration continues (step 612) until there is convergence, i.e. the error is approximately zero.

Once the concentrations of all the structures in solution are obtained (step 614) the algorithm records the information and is able to calculate the percentage amount relevant to these structures (step 616). If the percentage amount corresponds to 50% then the temperature of the reaction environment specified in the experimental conditions 104 corresponds to the effective Tm (step 618) .

It is remarkable that the temperature specified in the experimental conditions 104 together with the concentrations of Na+ and Mg++ ions serves as the basis for "in silico" simulation. As described above, starting from this information the algorithm is able to predict the conformation of the hairpins and dimers in solution in those specific reaction conditions, and then the melting temperature Tm and the effective melting temperature Tm.

It should be considered that the value of the melting temperature Tm is indicative but approximated, since it is based on the assumption that in solution there are only a selected primer and the DNA target sequence. With this assumption the $\Delta H$ and $\Delta S$ of primer-target dimer and starting concentrations of both species are sufficient for the calculation.

Since the calculation of the effective melting temperature takes into account that the reaction environment includes multiple combinations, and then the primer will bind not only to itself or to the target, but to all the other present species, the value of the effective melting temperature is considered more accurate and reliable than the simple melting temperature for the "in silico" simulation.

The algorithm described with reference to Figure 7 is able to predict all the concentrations of the structures (hairpins & dimers and species not hybridized, or random coil) at all temperature scale values. The temperature value in which the target DNA sequence is hybridized to the 50% to the primer (i.e. the concentration of the target-primer dimer will be equal to 50% of the target concentration) represents the effective temperature of melting.

The process starts again until the calculation has not been carried out for all temperature scale degrees (for example from 10 to 100 ° C). Once the process is complete, you can view the melting curves (step 620), which represents the trend of concentration of the structures with increasing temperature.

The calculation of the effective melting temperatures at different assay temperatures can be performed in two ways.

A first option (called FULL), necessarily slower, passes again to the prediction process 604, re-evaluating for each temperature degree the more stable structures through the hybridization of algorithms and folding.

A second option (called QUICK), more rapid, assumes that the secondary structures are always the same, as predicted the first time. Through this assumption the ΔG can be quickly computed (step 622), without the use of hybridization and folding algorithms, through the formula:

$$\Delta G_T^{\circ} = \Delta H^{\circ} - T\Delta S^{\circ}.$$

Once the ΔG values have been recalculated, the process continues as previously described.

In a preferred embodiment, the value of the effective melting temperature supports or replaces the value of the melting temperature in the design algorithms of the primers and of the primer sets. For example, the effective melting temperature value is used in place of, or in combination with the, 'simple' temperature of melting in the algorithms of calculation of the scores to be assigned to the primers and the primer sets.

Currently, there are no systems that are able to assess the formation of dimers of primers belonging to different primer sets in solution.

In its classic version initially described by Notomi, the LAMP technology is a method that allows rapid amplification of nucleic acids under isothermal conditions through the use of a DNA polymerase with strand-displacement activity and of four primers specifically designed to recognize six distinct regions of a target gene. By measurement of turbidimetry or fluorescence through the use of intercalating agents, the reaction of amplification of the single gene of interest (simplex) can be monitored in real time.

Over the years, however, this method has been modified and implemented: in particular through the introduction of fluorescent oligonucleotides, a technology was developed in which different transcripts of interest are amplified in the same tube in a single reaction and monitored in real-time through the use of specific fluorescent probes emitting at different wavelengths.

These modifications allow the development of duplex and triplex assays in which respectively one or two transcripts of interest are amplified together in an internal control, consisting of a housekeeping gene. For example, the PML-RARA essay consists of two different multiplex assays: a specific triplex assay for bcr1 and bcr3 fusion transcripts and a specific duplex essay for rarer bcr2 transcript.

In the case of multiplex reactions, the high number of primers inserted inside of the reaction mixture increases the probability of intermolecular interactions between the primers of different sets. For example, in the case of a triplex, the reaction mixture will contain a primer set for each of three different targets, i.e. up to a maximum total of 18 primers (12 if no loop primers are present). It is crucial, therefore, to have a tool that allows to evaluate not only the possible molecular interactions between primers of the same set, but above all the possible formation of dimers between the different sets of primers present in the solution.

This would allow to carry out a preliminary screening of the oligonucleotides already during the design phase, allowing to delete, modify or redesign potentially hazardous primers as prone to interact between them. It would also be useful to identify possible "difficult" sequences (for example palindromic regions or with a high rate of GC nucleotides) in order to avoid the design in such areas.

The evaluation of the formation of dimers between the different sets of primers allows to simulate, with more accuracy, the real conditions of interaction of the primers in solution in a multiplex reaction: reducing the gap between the "in silico" design predictions and the experimental results, the step of design and the next evaluation step of the primers in the reaction would be simpler, faster and less laborious.

The data and the formulas (calculation of Tm and salt influence in the solution) used as the basis for the calculation of the thermodynamic parameters can be found in the bibliography (SantaLucia, and J. Hicks, D., "The thermodynamics of DNA structural motifs", Annu. Rev. Biophys. Biomol. Struct. 2004. 33: 415-40).

For the evaluation and visualization of the secondary structures, the UNAFold algorithms (M. Zuker. Mfold web server for nucleic acid folding and hybridization prediction. Nucleic Acids Res. 31 (13), 3406-3415, 2003) have been used.

The primer design method according to the invention has been described for a reaction of amplification of a sequence of target DNA; However, it is evident to the skilled of the art that this method can also be applied for the amplification of a sequence of other nucleic acids.

**Claims**

1. Computer-based method for designing a set of primers to be used for an amplification reaction of a sequence of a target nucleic acid, comprising the steps of:

   a) providing a target nucleic acid sequence;

b) providing the conditions of the amplification reaction of the target nucleic acid sequence and design criteria of the primers, said design criteria comprising at least value ranges for the melting temperature, the ΔG limits of the hybridization between the end of a primer and the target nucleic acid sequence and hybridizations between the primers, and for the ΔG of the hairpin of a primer, the content in CG bases, and the distances between the primers composing the set;

c) manually selecting a candidate primer;

d) subjecting the primer to a hybridization algorithm to:

d1) predicting, from the multiple possible combinations between the candidate primer and the target nucleic acid sequence, the conformation of the most stable structure considering the values of ΔG, ΔH and ΔS;

d2) calculating from said values of ΔG, ΔH and ΔS the melting temperature between the candidate primer and the target nucleic acid sequence;

in the presence of at least one other previously selected candidate primer:

d3) predicting the conformation of the most stable of all the possible combinations of candidate primers considering the values of ΔG, ΔH and ΔS;

d4) calculating, from said values of ΔG, ΔH and ΔS, the concentration of all the structures present in the reaction environment;

d5) calculating, on the basis of said concentration value, the effective melting temperature between the candidate primer and the target sequence of nucleic acid, the effective melting temperature being the value of temperature in which the target nucleic acid sequence is 50% hybridized to the primer;

e) subjecting the candidate primer to a folding algorithm to predict the conformation of the most stable structure of the hairpin of the candidate primer and calculate the ΔG, ΔH and ΔS values thereof;

f) comparing said values obtained from the hybridization and folding algorithms with the design criteria;

g) if the result of said comparison is acceptable, repeating steps b)-e) with another candidate primer until a set of primers is obtained, otherwise

h) changing the selection of the candidate primer,

wherein, after selecting a set of primers, a step i) of graphical representation of the dumbbell on the computer screen is provided for, prior to the confirmation of acceptance of the primer set,

the method further comprising the steps of:

- manually selecting, on the graphical representation of the dumbbell, a new loop primer sequence in a position different from the one originally represented,
- consequently recalculating for said new loop primer sequence the values of the parameters corresponding to the design criteria,

wherein the monomeric dumbbell structure is predicted by means of the folding algorithm and represented graphically in order to permit the visualisation of any unexpected loops.

2. Method according to claim 1, wherein the step of selecting a candidate primer provides for displaying on a computer screen the target nucleic acid sequence and manually selecting the candidate primer on said sequence.

3. Method according to any of the preceding claims, wherein the design criteria comprise the length of the amplicon, and wherein, at the end of the selection of a set of primers, a step i) of calculating the length of the amplicon of the selected primer set and a step 1) of comparing the range of desired amplicon length and the length of the amplicon of the primer set selected, are provided for.

4. Method according to any of the preceding claims, further comprising a step of assigning a score to a selected primer, on the basis of the proximity of the predicted parameters, corresponding to the design criteria, to corresponding target values.

5. Method according to the preceding claim, further comprising a step of assigning a score to a selected set of primers, said score being calculated by means of the steps of:

- calculating a balancing score relative to the balancing of the melting temperature Tm of the pair of primers F3 and B3, F2 and B2, F1c and B1c, and possibly LF and LB, based on the proximity of the balance obtained from the predicted melting temperatures to a balance obtained from the design criteria,

- calculating a stability score relative to the stability ($\Delta G$) of the primer dimers using as predicted value the $\Delta G$ value of the most stable primer dimer, based on the proximity of the predicted value of $\Delta G$ to the corresponding design criterion,
- calculating the score of the primer set considering the contribution of the scores of the individual primers, of the balancing score and of the stability score.

6. Method according to the preceding claim, further comprising the step of calculating an amplicon score relative to the length of the amplicon, defined as the distance between the end of the F2 portion and the end of the B2 portion.

7. Method according to any of the preceding claims, wherein the steps d3) and d4) comprise the steps of:

i) calculating all the possible combinations of dimers (primer dimers or target primers) and monomers (random coil or hairpin) using the correct thermodynamic parameters database depending on the experimental conditions of the reaction environment and hybridization and folding algorithms;
ii) predicting the most stable secondary structures (dimers and hairpins) and providing the $\Delta G$ of such structures;
iii) calculating the equilibrium constant K according to the formula: $\Delta G = -RT * \ln(k)$;
iv) calculating all the concentrations of the structures in the reaction environment, for example by means of an iterative method;
v) repeating the above step at predetermined intervals of the temperature scale.

8. Method according to the preceding claim, wherein the step v) is performed by means of a repetition of steps i)-iv) .

9. Method according to claim 7, wherein the step v) is performed by re-calculating, for each temperature range, the $\Delta G$ using the formula $\Delta G_T° = \Delta H° - T \Delta S T°$.

10. Method according to any of the claims 7-9, comprising, after step iv), a calculation step of the percentage amounts relative to the concentrations of the structures in the reaction environment.

11. Method according to the preceding claim, wherein the actual melting temperature (actual Tm) of a structure is the temperature of the reaction environment at which the percentage amount of the concentration of the hybridized structure corresponds to 50%.

12. Computer program product, directly loadable in the memory of a computer, comprising software code portions suitable to implement the design method of a set of primers according to any of the preceding claims, when the computer program product is run on the computer.

## Patentansprüche

1. Computergestütztes Verfahren zum Entwerfen eines Satzes von Primern, die für eine Amplifikationsreaktion einer Sequenz einer Zielnukleinsäure zu verwenden sind, umfassend die Schritte:

a) Bereitstellen einer Zielnukleinsäuresequenz;
b) Bereitstellen der Bedingungen der Amplifikationsreaktion der Zielnukleinsäuresequenz und von Entwurfskriterien der Primer, wobei die Entwurfskriterien zumindest Wertebereiche für die Schmelztemperatur, die $\Delta G$-Grenzen der Hybridisierung zwischen dem Ende eines Primers und der Zielnukleinsäuresequenz und Hybridisierungen zwischen den Primern und für die $\Delta G$ der Haarnadel bzw. des Hairpin eines Primers, den Gehalt an CG-Basen und die Abstände zwischen den Primern, aus denen der Satz besteht, umfassen;
c) manuelles Auswählen eines potentiellen Primers bzw. Kandidaten-Primers;
d) Unterziehen des Primers einem Hybridisierungsalgorithmus zum:

d1) Vorhersagen, aus den mehreren möglichen Kombinationen zwischen dem Kandidaten-Primer und der Zielnukleinsäuresequenz, der Konformation der stabilsten Struktur unter Berücksichtigung der Werte von $\Delta G$, $\Delta H$ und $\Delta S$;
d2) Berechnen aus den Werten von $\Delta G$, $\Delta H$ und $\Delta S$ der Schmelztemperatur zwischen dem Kandidaten-Primer und der Zielnukleinsäuresequenz;
in Gegenwart von zumindest einem anderen zuvor ausgewählten Kandidaten-Primer:

d3) Vorhersagen der Konformation der stabilsten aller möglichen Kombinationen von Kandidaten-Primern unter Berücksichtigung der Werte von ∆G, ∆H und ∆S;

d4) Berechnen, aus den Werten von ∆G, ∆H und ∆S, der Konzentration aller in der Reaktionsumgebung vorhandenen Strukturen;

d5) Berechnen, auf der Basis des Konzentrationswerts, der effektiven Schmelztemperatur zwischen dem Kandidaten-Primer und der Zielnukleinsäuresequenz, wobei die effektive Schmelztemperatur der Temperaturwert ist, bei dem die Zielnukleinsäuresequenz zu 50 % zu bzw. mit dem Primer hybridisiert ist;

e) Unterziehen des Kandidaten-Primers einem Faltalgorithmus, um die Konformation der stabilsten Struktur der Haarnadel des Kandidaten-Primers vorherzusagen und dessen ∆G-, ∆H- und ∆S-Werte zu berechnen;

f) Vergleichen der aus den Hybridisierungs- und Faltungsalgorithmen erhaltenen Werte mit den Entwurfskriterien;

g) wenn das Ergebnis des Vergleichs akzeptabel ist, Wiederholen der Schritte b)-e) mit einem anderen Kandidaten-Primer, bis ein Satz von Primern erhalten wird, andernfalls

h) Ändern der Auswahl des Kandidaten-Primers, wobei nach der Auswahl eines Satzes von Primern ein Schritt i) der grafischen Darstellung der Hantel bzw. Dumbbell auf dem Computerbildschirm vorgesehen ist, und zwar vor der Bestätigung der Akzeptanz des Satzes von Primern,

wobei das Verfahren ferner die Schritte umfasst:

- manuelles Auswählen, auf der grafischen Darstellung der Hantel, einer neuen Loop- bzw. Schleifen-Primer-Sequenz in einer von der ursprünglich dargestellten unterschiedlichen Position,
- anschließendes Neuberechnen der Werte der Parameter, die den Entwurfskriterien entsprechen, für die neue Schleifen-Primer-Sequenz,

wobei die monomere Hantelstruktur mittels des Faltungsalgorithmus vorhergesagt und grafisch dargestellt wird, um die Visualisierung etwaiger unerwarteter Schleifen zu ermöglichen.

2. Verfahren nach Anspruch 1, wobei der Schritt des Auswählens eines Kandidaten-Primers ein Anzeigen der Zielnukleinsäuresequenz auf einem Computerbildschirm und ein manuelle Auswählen des Kandidaten-Primers auf dieser Sequenz vorsieht.

3. Verfahren nach einem der vorhergehenden Ansprüche, wobei die Entwurfskriterien die Länge des Amplikons umfassen und wobei am Ende der Auswahl eines Satzes von Primern ein Schritt i) des Berechnens der Länge des Amplikons des ausgewählten Satzes von Primern und ein Schritt 1) des Vergleichens des Bereichs der gewünschten Amplikonlänge und der Länge des Amplikons des ausgewählten Satzes von Primern vorgesehen sind.

4. Verfahren nach einem der vorhergehenden Ansprüche, ferner umfassend einen Schritt des Zuweisens eines Scores zu einem ausgewählten Primer auf der Basis der Nähe der den Entwurfskriterien entsprechenden vorhergesagten Parametern zu entsprechenden Zielwerten.

5. Verfahren nach dem vorhergehenden Anspruch, ferner umfassend einen Schritt des Zuweisens eines Scores zu einem ausgewählten Satz von Primern, wobei der Score mittels der folgenden Schritte berechnet wird:

- Berechnen eines Balance-Scores relativ zu der Balance der Schmelztemperatur Tm des Primer-Paars F3 und B3, F2 und B2, F1c und B1c und möglicherweise LF und LB, basierend auf der Nähe der aus den vorhergesagten Schmelztemperaturen erhaltenen Balance zu einer aus den Entwurfskriterien erhaltenen Balance,
- Berechnen einer Stabilitäts-Scores relativ zu der Stabilität (∆G) der Primer-Dimere unter Verwendung des ∆G-Werts des stabilsten Primer-Dimers als vorhergesagten Wert, basierend auf der Nähe des vorhergesagten ∆G-Werts zum dem entsprechenden Entwurfskriterium,
- Berechnen des Scores des Satzes von Primern unter Berücksichtigung des Beitrags der Scores der einzelnen Primer, des Balance-Scores und des Stabilitäts-Scores.

6. Verfahren nach dem vorhergehenden Anspruch, ferner umfassend den Schritt des Berechnens eines Amplikon-Scores relativ zu der Länge des Amplikons, die als der Abstand zwischen dem Ende des F2-Abschnitts und dem Ende des B2-Abschnitts definiert ist.

7. Verfahren nach einem der vorhergehenden Ansprüche, wobei die Schritte d3) und d4) die Schritte umfassen:

i) Berechnen aller möglichen Kombinationen von Dimeren (Primer-Dimere oder Ziel-Primer) und Monomeren (Random Coil oder Hairpin bzw. Haarnadel) unter Verwendung der korrekten Datenbank thermodynamischer Parameter in Abhängigkeit von den experimentellen Bedingungen der Reaktionsumgebung und den Hybridisierungs- und Faltungsalgorithmen;

ii) Vorhersagen der stabilsten Sekundärstrukturen (Dimere und Hairpins bzw. Haarnadeln) und Bereitstellen des ΔG solcher Strukturen;

iii) Berechnen der Gleichgewichtskonstante K gemäß der Formel: $\Delta G = -RT * \ln(k)$;

iv) Berechnen aller Konzentrationen der Strukturen in der Reaktionsumgebung, beispielsweise mittels einer iterativen Methode;

v) Wiederholen des obigen Schritts in vorbestimmten Intervallen der Temperaturskala.

8. Verfahren nach dem vorhergehenden Anspruch, wobei der Schritt v) mittels einer Wiederholung der Schritte i)-iv) durchgeführt wird.

9. Verfahren nach Anspruch 7, wobei der Schritt v) durch Neuberechnung des ΔG für jeden Temperaturbereich unter Verwendung der Formel $\Delta G_T° = \Delta H° - T\Delta S T°$ durchgeführt wird.

10. Verfahren nach einem der Ansprüche 7-9, umfassend nach Schritt iv) einen Berechnungsschritt der Prozentbeträge relativ zu den Konzentrationen der Strukturen in der Reaktionsumgebung.

11. Verfahren nach dem vorhergehenden Anspruch, wobei die tatsächliche Schmelztemperatur (tatsächliche Tm) einer Struktur die Temperatur der Reaktionsumgebung ist, bei der der Prozentbetrag der Konzentration der hybridisierten Struktur 50 % entspricht.

12. Computerprogrammprodukt, das direkt in den Speicher eines Computers ladbar ist, umfassend Softwarecodeabschnitte, die geeignet sind, das Entwurfsverfahren eines Satzes von Primern nach einem der vorhergehenden Ansprüche zu implementieren, wenn das Computerprogrammprodukt auf dem Computer ausgeführt wird.

**Revendications**

1. Procédé informatique pour concevoir un ensemble d'amorces à utiliser pour une réaction d'amplification d'une séquence d'un acide nucléique cible, comprenant les étapes suivantes :

   a) la fourniture d'une séquence d'acide nucléique cible ;

   b) la fourniture des conditions de la réaction d'amplification de la séquence d'acide nucléique cible et des critères de conception des amorces, lesdits critères de conception comprenant au moins des plages de valeurs pour la température de fusion, les limites ΔG de l'hybridation entre l'extrémité d'une amorce et la séquence d'acide nucléique cible et des hybridations entre les amorces, et pour le ΔG de l'épingle à cheveux d'une amorce, la teneur en bases CG, et les distances entre les amorces composant l'ensemble ;

   c) la sélection manuellement d'une amorce candidate ;

   d) la soumission de l'amorce à un algorithme d'hybridation pour :

      d1) la prédiction, à partir des multiples combinaisons possibles entre l'amorce candidate et la séquence d'acide nucléique cible, de la conformation de la structure la plus stable en tenant compte des valeurs de ΔG, ΔH et ΔS ;

      d2) le calcul, à partir desdites valeurs de ΔG, ΔH et ΔS, de la température de fusion entre l'amorce candidate et la séquence d'acide nucléique cible ;

      en présence d'au moins une autre amorce candidate précédemment sélectionnée :

         d3) la prédiction de la conformation de la plus stable de toutes les combinaisons possibles d'amorces candidates en tenant compte des valeurs de ΔG, ΔH et ΔS ;

         d4) le calcul, à partir desdites valeurs de ΔG, ΔH et ΔS, de la concentration de toutes les structures présentes dans l'environnement de réaction ;

         d5) le calcul, sur la base de ladite valeur de concentration, de la température de fusion efficace entre l'amorce candidate et la séquence d'acide nucléique cible, la température de fusion efficace étant la valeur de température à laquelle la séquence d'acide nucléique cible est hybridée à 50 % à l'amorce ;

e) la soumission de l'amorce candidate à un algorithme de repliement pour prédire la conformation de la structure la plus stable de l'épingle à cheveux de l'amorce candidate et calculer les valeurs de ΔG, ΔH et ΔS de celle-ci ;
f) la comparaison desdites valeurs obtenues à partir des algorithmes d'hybridation et de repliement aux critères de conception ;
g) si le résultat de ladite comparaison est acceptable, la répétition des étapes b) à e) avec une autre amorce candidate jusqu'à ce qu'un ensemble d'amorces soit obtenu, sinon
h) le changement de la sélection de l'amorce candidate,
dans lequel, après la sélection d'un ensemble d'amorces, une étape i) de représentation graphique de l'haltère sur l'écran informatique est prévue, avant la confirmation d'acceptation de l'ensemble d'amorces,
le procédé comprenant en outre les étapes suivantes :

- la sélection manuellement, sur la représentation graphique de l'haltère, d'une nouvelle séquence d'amorce en boucle dans une position différente de celle initialement représentée,
- le recalcul, en conséquence de ladite nouvelle séquence d'amorce en boucle, des valeurs des paramètres correspondant aux critères de conception,

dans lequel la structure d'haltère monomère est prédite au moyen de l'algorithme de repliement et représentée graphiquement afin de permettre la visualisation de toute boucle imprévue.

2. Procédé selon la revendication 1, dans lequel l'étape de la sélection d'une amorce candidate prévoit l'affichage, sur un écran informatique, de la séquence d'acide nucléique cible et la sélection manuellement de l'amorce candidate sur ladite séquence.

3. Procédé selon l'une quelconque des revendications précédentes, dans lequel les critères de conception comprennent la longueur de l'amplicon, et dans lequel, à la fin de la sélection d'un ensemble d'amorces, une étape i) de calcul de la longueur de l'amplicon de l'ensemble d'amorces sélectionné et une étape 1) de comparaison de la plage de la longueur d'amplicon souhaitée et de la longueur de l'amplicon de l'ensemble d'amorces sélectionné sont prévues.

4. Procédé selon l'une quelconque des revendications précédentes, comprenant en outre une étape d'attribution d'un score à une amorce sélectionnée, sur la base de la proximité des paramètres prédits, correspondant aux critères de conception, des valeurs cibles correspondantes.

5. Procédé selon la revendication précédente, comprenant en outre une étape d'attribution d'un score à un ensemble sélectionné d'amorces, ledit score étant calculé au moyen des étapes suivantes :

- le calcul d'un score d'équilibrage en ce qui concerne l'équilibrage de la température de fusion Tm de la paire d'amorces F3 et B3, F2 et B2, F1c et B1c, et éventuellement LF et LB, sur la base de la proximité de l'équilibre obtenu à partir des températures de fusion prédites d'un équilibre obtenu à partir des critères de conception,
- le calcul d'un score de stabilité en ce qui concerne la stabilité (ΔG) des dimères d'amorce en utilisant, comme valeur prédite, la valeur de ΔG du dimère d'amorce le plus stable, sur la base de la proximité de la valeur prédite de ΔG du critère de conception correspondant,
- le calcul du score de l'ensemble d'amorces en tenant compte de la contribution des scores des amorces individuelles, du score d'équilibrage et du score de stabilité.

6. Procédé selon la revendication précédente, comprenant en outre l'étape de calcul d'un score d'amplicon en ce qui concerne la longueur de l'amplicon, définie comme la distance entre l'extrémité de la partie F2 et l'extrémité de la partie B2.

7. Procédé selon l'une quelconque des revendications précédentes, dans lequel les étapes d3) et d4) comprennent les étapes suivantes :

i) le calcul de toutes les combinaisons possibles de dimères (dimères d'amorces ou amorces cibles) et de monomères (bobine ou épingle à cheveux aléatoire) en utilisant la base de données de paramètres thermodynamiques corrects en fonction des conditions expérimentales de l'environnement de réaction et des algorithmes d'hybridation et de repliement ;
ii) la prédiction des structures secondaires les plus stables (dimères et épingles à cheveux) et la fourniture du ΔG de telles structures ;

iii) le calcul de la constante d'équilibre K selon la formule :

$$\Delta G = -RT * \ln (k) \ ;$$

iv) le calcul de toutes les concentrations des structures dans l'environnement de réaction, par exemple au moyen d'un procédé itératif ;
v) la répétition de l'étape précédente à des intervalles prédéterminés de l'échelle de température.

8. Procédé selon la revendication précédente, dans lequel l'étape v) est réalisée au moyen d'une répétition des étapes i) à iv).

9. Procédé selon la revendication 7, dans lequel l'étape v) est réalisée par le recalcul, pour chaque plage de températures, du $\Delta G$ en utilisant la formule $\Delta G_T° = \Delta H° - T\Delta ST°$.

10. Procédé selon l'une quelconque des revendications 7 à 9, comprenant, après l'étape iv), une étape de calcul des quantités de pourcentage en ce qui concerne les concentrations des structures dans l'environnement de réaction.

11. Procédé selon la revendication précédente, dans lequel la température de fusion réelle (Tm réelle) d'une structure est la température de l'environnement de réaction à laquelle la quantité en pourcentage de la concentration de la structure hybridée correspond à 50 %.

12. Produit programme informatique, pouvant être directement chargé dans la mémoire d'un ordinateur, comprenant des parties de code logiciel adaptées pour mettre en oeuvre le procédé de conception d'un ensemble d'amorces selon l'une quelconque des revendications précédentes, lorsque le produit programme informatique est exécuté sur l'ordinateur.

FIG.1

EP 3 394 777 B1

FIG.2

FIG.3

FIG.4

FIG.5

FIG.6

FIG.6a

FIG.7

FIG. 8

FIG. 8a

EP 3 394 777 B1

704

$$Tm_{target} = (Tm_{max} + Tm_{min}) / 2$$

$$Tm_{tolerance} = (Tm_{max} - Tm_{min}) / 2$$

$$Tm_{score} = 1 - \frac{Abs(Tm_{predicted} - Tm_{target})}{Tm_{tolerance}} * 100$$

$$Tm_{max} = 61 \quad Tm_{min} = 63 \quad Tm_{predicted} = 61.85$$

$$Tm_{score} = 1 - \frac{Abs(61.85 - 62)}{1} * 100 = 85\%$$

FIG.9

FIG.10

FIG.11

Length

| 20 | 22 |
| 18 | 20 |
| 18 | 20 |
| 15 | 20 |

GC Rate

| 40 | 65 |

F1c / B1c
F2 / B2
F3 / B3
LF / LB

Tm

| 64 | 66 |
| 59 | 61 |
| 59 | 61 |
| 60 | 66 |

ID **329**

dG Threshold

Inner/Outer Primers
5' stability    1.3
3' stability    0.7
Dimer check   -2.1

Loop Primers
3' stability    2
Dimer check   -3.2

CG clamp (min)    0

Preset Defaults (Length, Tm and CG rate)

AT rich    Normal    GC rich

Current Target GC content i    0.0 (AT rich)

Design Criteria

**FIG.12**

Assisted Design

ID 341

CAAGTTGCCGCTAATCAGAAATAAATTCATTGCAACGTTAAATACATCACAAAATGGCCGCTCAAAAACAGGCTTGAACATATAATGACTGCCTCTCATTC
TCTGTTTCATTTTTCACTCCCATATTATAGGGTGAAATATAATCGTGTATGCGAGAGTAGTGGCAACATATTGTGCTCTTCGATTTTTTGGCAACCCAAATT
GGAGGCAGACGAACGAGATGATACCATATGCAAATTGTAGCTAATTTTTCTCTCCCATATATATAAGACCGAAAACGGCTGTTTAAAAGTTCCCAATAGATT
GCCAATATTTTAAGTCTATATATTTCTGAGAATAAATTGACCAGATTAAATATTAAAGGCCATGTTTTTGTATTGCTTTTTTTTATACCCCTTACTCATATAT
TTAAAGGGGTACACTATATTCGTTGAAGAGTATGTAACAAACAGTATGAAATGTCTCCGACTATATATAAGTATATATATTTTTGATCATATATAAGGCCG
AGGCCGATCTAGCCATGTCCGTC░░░░░░░░░░░░░░░░░░░░GATCTCAAGAACTATAAAATCAA░░░░░░░░░░░░░░ATATAGATTCTAGAGACA
AGGACGCAGTGCAAGTTTGGTGACCCATGTTGTCACGCCCCATTCTAACGTCC░░░░░░░░░░░░GCCATGCCCACATTTTTAAACCATTTTTTCCTA
TTTTTTTTCACATTTTTATTAACTTTTGTAAATTTCTATCACTCTTCAAAAAAAAAATTTGCCATGCCCACATTTTTTAACCATTCTTCGATA

| F3 | F2 | | | | B1c | L8 | | | 3' | Working on: F2 | | Score 42.4 |

3' | | | LF | F1c | | | | B2 | B3 |

Solution Conditions K = 62.0  NaCl = 0.050 M  MgCl = 0.005 M  Glycerol % = 0.0

Primers

| Name | | Pos | Len | 5' Sequence | CG% | End dG | Cl | Conc M | Tm | i | Score | Info |
|------|---|-----|-----|-------------|-----|--------|----|--------|----|----|-------|------|
| F3 | - | 897 | 18 | taatcaaaagccGAGGCG | 50.0 | -2.1 | 4 | 2E-07 | 60.6 | % | 55.8 | |
| B3 | - | 1063 | 18 | cagttttgtgcgGTTTGT | 44.4 | -0.1 | 1 | 2E-07 | 60.4 | % | 37.1 | |
| F2 | xyz | 932 | 19 | tgttcgtatgaacGTCGAG | 47.4 | -0.8 | 2 | 1.6E-06 | 63.3 | a | 34.6 | |
| F1c | - | 973 | 20 | GCTGCTtaatctcaaccttc | 45.0 | -2.5 | 2 | 1.6E-06 | 64.7 | % | 66.1 | |
| B2 | - | 1040 | 18 | ttagaatgggcgTGACAA | 44.4 | -0.4 | 1 | 1.6E-06 | 60.4 | % | 41.6 | |
| B1c | - | 998 | 20 | GATTCTagagacaaggacgc | 50.0 | 0.2 | 1 | 1.6E-06 | 63.0 | % | 63.8 | |
| LF | - | | | | | | | 8E-07 | | | 0.0 | |
| L8 | - | | | | | | | 8E-07 | | | 0.0 | |
| | | 931 | 20 | ctgttcgtatgaacGTCGAG | 50.0 | -2.0 | 2 | 1.6E-06 | 67.4 | a | 44.6 | (F2) |

Primers

| | F3 | B3 | | B2 | B1c | LF | L8 | | homo | hpin |
|---|----|----|----|----|-----|----|----|---|------|------|
| F3 > | -0.7 | 0.0 | -0.3 | -1.1 | -0.9 | | | 0.0 | -0.9 | |
| B3 > | | +0.2 | +0.2 | 0.0 | -1.1 | | | +0.2 | 0.0 | |
| F2 > | | | +0.7 | -0.2 | -1.1 | | | 0.0 | 0.0 | |
| | | | | +0.4 | -0.1 | | | +0.7 | +1.0 | |
| B2 > | | | | | -1.1 | | | -0.2 | +0.6 | |
| B1c > | | | | | | | | -1.1 | +0.3 | |
| LF > | | | | | | | | | | |
| L8 > | | | | | | | | | 0.0 | |

Secondary Structures

FIG. 13

FIG.14

FIG. 15

FIG. 16

FIG. 17

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. NOTOMI ; H. OKAYAMA ; H. MASUBUCHI et al.** Loop-mediated isothermal amplification of DNA. *Nucleic Acids Research,* 2000, vol. 28 (12
- **SANTALUCIA ; J. HICKS, D.** The thermodynamics of DNA structural motifs. *Annu. Rev. Biophys. Biomol. Struct.,* 2004, vol. 33, 415-40
- **SANTALUCIA ; J. HICKS, D.** The thermodynamics of DNA structural motifs. *Annu. Rev. Biophys. Biomol. Struct .,* 2004, vol. 33, 415-40
- **M. ZUKER.** Mfold web server for nucleic acid folding and hybridization prediction. *Nucleic Acids Res.,* 2003, vol. 31 (13), 3406-3415
- SantaLucia Hicks. *THE THERMODYNAMICS OF DNA STRUCTURALMOTIFS,* 2004, 419
- **ZERILLI et al.** *Clin Chem,* 2010, 1287-96
- **M. ZUKER.** Mfold web server for nucleic acid folding and hybridization prediction. *Nucleic Acids Res.,* 2003, vol. 31 (13), 3406-3415